# EUROPÄISCHE PATENTSCHRIFT

(11) **EP 3 223 790 B1**
(45) Veröffentlichungstag und Bekanntmachung des Hinweises auf die Patenterteilung: **27.05.2020**
(21) Anmeldenummer: 15784308.7
(22) Anmeldetag: 14.10.2015
(51) Int. Cl.: A61K 8/49, A61Q 5/06

(54) **MITTEL ZUM FÄRBEN VON KERATINISCHEN FASERN, ENTHALTEND MINDESTENS EINEN DIMEREN, DIKATIONISCHEN AZOFARBSTOFF MIT SPEZIELLEM SUBSTITUTIONSMUSTER**
AGENTS FOR DYEING KERATIN FIBRES, CONTAINING AT LEAST ONE DIMERIC, DICATIONIC AZO DYE WITH SPECIAL SUBSTITUTION PATTERN
AGENTS DE COLORATION DE FIBRES KÉRATINIQUES CONTENANT AU MOINS UN COLORANT AZOÏQUE DICATIONIQUE DIMÈRE AYANT UN MOTIF DE SUBSTITUTION PARTICULIER

(30) Priorität: 25.11.2014 DE 102014223936
(43) Veröffentlichungstag der Anmeldung: 04.10.2017
(73) Patentinhaber: Henkel AG & Co. KGaA, 40589 Düsseldorf (DE)
(72) Erfinder: GEBERT-SCHWARZWÄLDER, Antje, 41469 Neuss (DE); GIESA, Helmut, 40670 Meerbusch (DE); KROOS, Astrid, 40789 Monheim (DE)
(86) Internationale Anmeldenummer: PCT/EP2015/073779
(87) Internationale Veröffentlichungsnummer: WO 2016/083015

(56) Entgegenhaltungen:
- EP-A1- 1 448 156
- EP-A2- 1 416 908
- EP-A2- 1 483 334
- DE-A1- 2 822 912
- FR-A1- 2 915 681
- US-A- 4 607 071
- US-A1- 2004 200 009

## Beschreibung

Die vorliegende Erfindung betrifft Mittel zum Färben von keratinischen Fasern, insbesondere menschlichen Haaren, die (a) mindestens einen dimeren, dikationischen Azofarbstoff einer speziellen Formel (I) enthalten.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen.

Abhängig vom gewünschten Farbergebnis setzt der Fachmann direktziehende Farbstoffe unterschiedlicher Farbstoffklassen ein. Die aus dem Stand der Technik bekannten direktziehenden Farbstoffe gehören beispielsweise zur Klasse der Nitrofarbstoffe, der Anthrachinonfarbstoffe, der Azofarbstoffe, der Triarylmethanfarbstoffe oder der Methinfarbstoffe. Alle diese Farbstoffklassen sollten für die Anwendung im Kosmetikbereich ein bestimmtes Anforderungsprofil erfüllen. So sollten direktziehende Farbstoffe ein intensives Färbeergebnis liefern und möglichst gute Echtheitseigenschaften besitzen. Durch Umwelteinflüsse sollte das mit direktziehenden Farbstoffen erhaltene Farbergebnis möglichst wenig beeinflusst werden, d.h. die Farbstoffe sollten beispielsweise eine gute Waschechtheit, Lichtechtheit und Reibechtheit besitzen. Auch chemische Einflüsse, welchen die keratinischen Fasern nach dem Färbeprozess ausgesetzt sein können (wie beispielsweise Dauerwellen), sollten das Farbergebnis möglichst wenig verändern.

Um gleichzeitig mit der Färbung auch eine Aufhellung zu erreichen, sollten die direktziehenden Farbstoffe nach Möglichkeit auch mit den bei Blondierverfahren üblicherweise eingesetzten Oxidationsmitteln (wie z.B. Wasserstoffperoxid und/oder Persulfate) kompatibel sein.

Für die starke Aufhellung von dunklen Haaren wird nicht nur Wasserstoffperoxid allein, sondern eine Kombination aus Wasserstoffperoxid und Persulfaten (z.B. Ammoniumpersulfat, Kaliumpersulfat und/oder Natriumpersulfat) eingesetzt. Soll also dunkles Haar in einem Schritt stark aufgehellt und gleichzeitig in einem leuchtenden Farbton gefärbt werden, ist der Einsatz einer Mischung aus Wasserstoffperoxid, Persulfaten und einem direktziehenden Farbstoff von Vorteil. Obwohl dem Fachmann zum Färben von Haaren viele intensiv färbende direktziehende Farbstoffe bekannt sind, so kennt er doch nur eine sehr begrenzte Auswahl an Farbstoffen, die die starken oxidativen Bedingungen, wie sie eine Mischung der oben genannten Oxidationsmittel darstellt, ohne Zersetzung überstehen. Zudem weisen die aus dem Stand der Technik bekannten oxidationsstabilen Farbstoffe im Hinblick auf ihre übrigen Echtheitseigenschaften gravierende Nachteile auf.

Für das gleichzeitige Färben und starke Aufhellen von Haaren besteht damit nach wie vor ein Bedarf an Farbstoffen mit hoher Stabilität gegenüber starken Oxidationsmitteln. Auch unter diesen extremen Anwendungsbedingungen sollen diese Farbstoffe ihre positiven Echtheits- und Färbeeigenschaften nicht verlieren.

Es hat sich gezeigt, dass leuchtende und intensive Färbungen insbesondere mit kationischen direktziehenden Farbstoffen erzeugt werden können. Kationische Farbstoffe zeichnen sich oft durch eine besonders hohe Affinität zu Keratinfasern aus, was auf die Wechselwirkungen der positiven Ladungen der Farbstoffe mit negativ geladenen Strukturbestandteilen der keratinischen Fasern zurückgeführt werden kann. Daher lassen sich mit kationischen Farbstoffen oftmals besonders intensive Färbungen erzielen.

Aus dem Stand der Technik hinlänglich bekannte monomere kationische Azofarbstoffe sind beispielsweise die Vertreter Basic Orange 31 (Alternativname: 2-[(4-Aminophenyl)azo]-1 ,3-dimethyl-1 H-imidazolium chloride, CAS-Nr. 97404-02-9) und Basic Red 51 (Alternativname: 2-[((4-Dimethylamino)phenyl)azo]-1,3-dimethyl-1H-imidazolium chlorid, CAS-Nr. 77061-58-6). US2004/200009 offenbart dimere dikationische direktziehende Farbstoffe.

Beide Farbstoffe färben keratinischen Fasern mit herausragender Farbintensität in orange bis roten Nuancenbereich. Es besteht weiterhin auch noch Bedarf an direktziehenden Farbstoffen, welche mit diesen beiden Farbstoffen in optimaler Weise kompatibel sind.

Aufgabe der vorliegenden Anmeldung ist es daher, Färbemittel für keratinische Fasern, insbesondere menschliche Haare, bereitzustellen, die hinsichtlich der Farbtiefe und der Echtheitseigenschaften, wie insbesondere Licht-, Reib- und Waschechtheit sowie Schweiß- und Kaltwellechtheit, gute anwendungstechnische Eigenschaften besitzen. Im Falle der gleichzeitigen Anwendung mit Oxidationsfarbstoffen und/oder Oxidationsmitteln sollen die direktziehenden Farbstoffe eine hohe Stabilität gegen Wasserstoffperoxid und andere Oxidationsmittel aufweisen und ihre positiven Echtheits- und Färbeeigenschaften nicht verlieren. Zudem sollten möglichst leuchtende und intensive Färbungen erzielt werden.

Darüber hinaus sollen die vorgenannten Farbstoffe auch besonders gut mit den kationischen Azofarbstoffen Basic Orange 31 und Basic Red 51 kompatibel sein.

Es konnte nun gefunden werden, dass die Anwendung von dimeren dikationischen Azofarbstoffen der allgemeinen Formel (I) auf keratinischen Fasern zu intensiven und leuchtenden Färbungen führt. Überraschenderweise zeichneten sich die direktziehenden Farbstoffe der allgemeinen Formel (I) auch durch eine hohe Stabilität gegenüber Oxidationsmitteln aus. Auf diesem Wege wurde durch den gleichzeitigen Einsatz von mindestens einem Farbstoff der Formel (I), Wasserstoffperoxid und einem Persulfatsalz die simultane Aufhellung (Bleiche) und Färbung möglich.

Ein erster Gegenstand der vorliegenden Erfindung ist ein Mittel zur Farbveränderung von keratinischen Fasern, insbesondere menschlichen Haaren, enthaltend in einem kosmetischen Träger (a) mindestens einen direktziehenden Farbstoff der Formel (I), 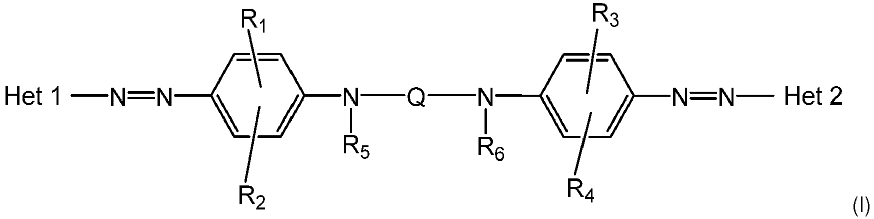 wobei
- Het1, Het2: unabhängig voneinander für eine der Strukturen (II), (III), (IV), (V), (VI) oder (VII) stehen, 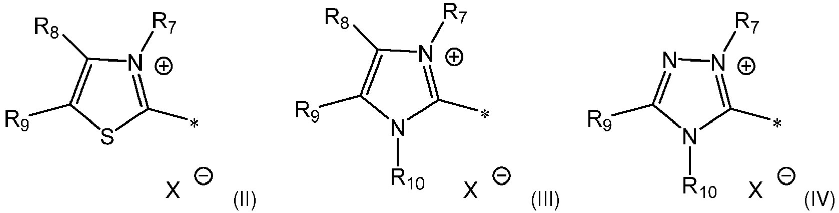 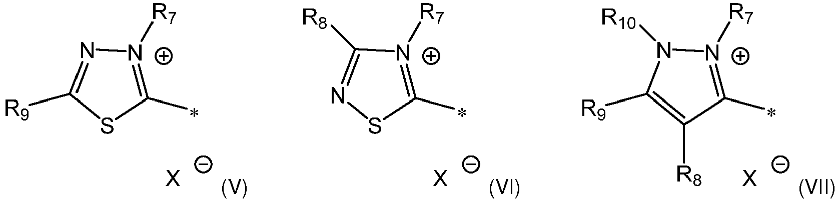
- R1, R3: unabhängig voneinander für eine C₁-C₆-Alkylgruppe, oder für eine C₁-C₆-Alkoxygruppe stehen,
- R2, R4: beide für ein Wasserstoffatom, stehen,
- R5, R6: unabhängig voneinander für ein Wasserstoffatom, eine C₁-C₆-Alkylgruppe oder für eine C₂-C₆-Alkenylgruppe stehen,
- Q: für eine Gruppierung der Formel (VIII) steht,

*-(CH₂)n-* (VIII)
- n: für eine ganze Zahl von 3 bis 6 steht,

- R7, R10: jeweils unabhängig voneinander für eine C₁-C₆-Alkylgruppe oder für eine C₂-C₆-Alkenylgruppe stehen,
- R8, R9: jeweils unabhängig voneinander für ein Wasserstoffatom, eine C₁-C₆-Alkyl-gruppe, ein Halogenatom aus der Gruppe Chlor, Brom, Fluor oder Jod, für eine C₁-C₆-Alkoxygruppe oder für eine Nitrogruppe stehen,
- X-: für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat, steht.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Farbveränderung von Keratinfasern" umfasst jedwede Form der Farbveränderung der Fasern. Umfasst sind insbesondere die unter den Begriffen Tönung, Blondierung, Mattierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen. Unter einer färbenden Blondierung wird die simultane Aufhellung und Färbung der keratinischen Fasern verstanden, die durch den gleichzeitigen Einsatz von Oxidationsmittel und Farbstoff im Färbänderungsmittel erzielt werden kann.

Die erfindungsgemäßen Mittel enthalten die direktziehenden Farbstoffe der Formel (I) in einem kosmetischen Träger. Dieser kosmetische Träger ist bevorzugt wässrig, alkoholisch oder wässrigalkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als wesentlichen Inhaltsstoff (a) enthalten die erfindungsgemäßen Mittel mindestens einen direktziehenden Farbstoff der Formel (I).

Die Substituenten R1 bis R10 der Verbindungen der Formel (I) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Halogenatome sind ausgewählt aus der Gruppe Chlor, Brom, Fluor und/oder Jod, hierbei sind Chlor und Brom besonders bevorzugt. Beispielhaft für eine C₁-C₆-Alkoxygruppe können die Methoxy-, die Ethoxy- und die Propoxygruppe genannt werden. Unter einer Nitrogruppe ist eine Gruppierung -NO₂ zu verstehen.

Jeder direktziehende Farbstoff der Formel (I) trägt zwei kationische, heterozyklische Endgruppen Het 1 und Het 2, die unabhängig voneinander aus den Gruppierungen der Formeln (II) bis (VII) ausgewählt sind

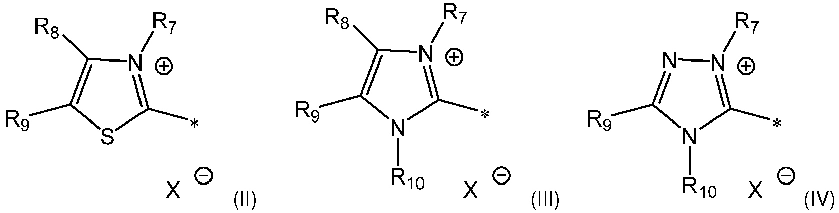

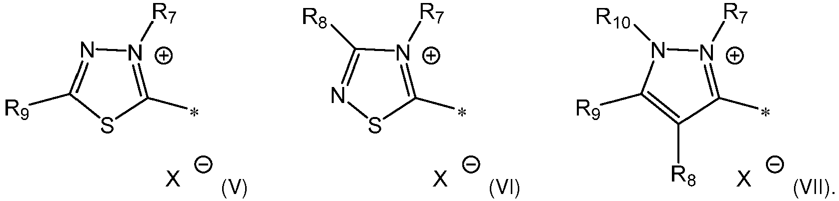

Formel (II) stellt eine kationische Thiazoliumendgruppe dar.

Formel (III) stellt eine kationische Imidazoliumendgruppe dar.

Formel (IV) stellt eine kationische, 1,2,4-Triazoliumendgruppe dar.

Formel (V) stellt eine kationische 1,3,4-Thiadiazoliumendgruppe dar.

Formel (VI) stellt eine kationische 1,2,4-Thiadiazoliumendgruppe dar.

Formel (VII) stellt eine kationische Pyrazoliumendgruppe dar.

In den Formeln (II) bis (VII) ist jeweils eine Position mit einem Stern markiert, welche jeweils die Verknüpfungsstelle der heterozyklischen Endgruppe Het 1 bzw. Het 2 mit der Azo-Gruppe in der Formel (I) angibt.

Die heterozyklischen Endgruppen Het 1 und Het 2 können in den Farbstoffen der Formel (I) gleich oder verschieden sein. Reinere Färbungen werden erhalten, wenn Het 1 und Het 2 in einem Farbstoff der Formel (I) gleich sind.

Durch die Auswahl verschiedener Endgruppen Het 1 und Het 2 kann das Absorptionsspektrum des Farbstoffes und demnach auch die Färbung des Farbstoffes beeinflusst werden.

Wenn intensive Färbungen im Violettbereich oder Blauviolettbereich gewünscht werden, ist es von Vorteil, wenn Het 1 und Het 2 für heterozyklische Endgruppen der Formel (II) stehen.

Sollen brillante Färbungen im Rotbereich erzielt werden, ist es hingegen bevorzugt, wenn Het 1 und Het 2 aus den heterozyklischen Endgruppen der Formeln (III) und/oder (IV) ausgewählt werden.

Darüberhinaus lassen sich mit Farbstoffen der Formel (I), bei welchen Het 1 und Het 2 für eine Gruppe ausgewählt aus den Formeln (II), (III) oder (IV) steht, besonders reine und leuchtende Farbtöne erzeugen. Aus diesem Grund ist es besonders bevorzugt, wenn das erfindungsgemäße Mittel (a) mindestens einen direktziehenden Farbstoff der allgemeinen Formel (I) enthält, in der
Het1, Het2 unabhängig voneinander für eine der Strukturen (II), (III) oder (IV) stehen.

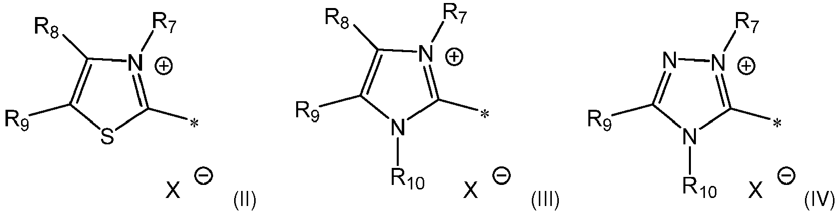

In einer besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
Het1, Het2 unabhängig voneinander jeweils für eine der Strukturen (II), (III) oder (IV) stehen,

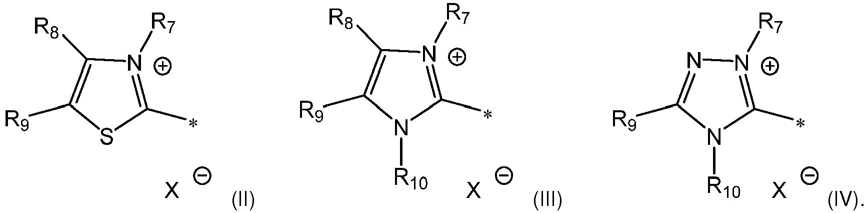

Die Reste R1 bis R4 geben die Substituenten an den beiden Phenylringen des direktziehenden Farbstoffes der Formel (I) an.

Hierbei können R1 und R3 unabhängig voneinander für eine C₁-C₆-Alkylgruppe, oder für eine C₁-C₆-Alkoxygruppe stehen. Prinzipiell können R1 und R3 in dem Farbstoff der Formel (I) gleich oder verschieden sein. Es ist jedoch bevorzugt wenn R1 und R3 in jedem Farbstoff der Formel (I) gleich sind.

Die Reste R2 und R4 stehen beide für ein Wasserstoffatom. Überraschenderweise hat sich herausgestellt, dass die Anwesenheit von mindestens einem Substituenten an jedem der beiden Phenylringe eines Farbstoffes der Formel (I) ganz wesentlichen Einfluss die Oxidationsstabilität des Farbstoffes nimmt.

Wird ein dimerer Azofarbstoff vom prinzipiellen Aufbau der Formel (I), der jedoch am Phenylring unsubstituiert ist (mit R1 bis R4 gleich Wasserstoff), in Kombination mit Wasserstoffperoxid und Persulfatsalzen auf Keratinfasern ausgefärbt, so können die Keratinfasern nur in blassen Nuancen gefärbt werden.

Wird für die aufhellende Färbung jedoch ein erfindungsgemäßer Farbstoff der Formel (I) (mit R1 und R3 ungleich Wasserstoff) in Kombination mit Wasserstoffperoxid und Persulfatsalzen eingesetzt, so ist die Intensität des Farbergebnisses deutlich intensiver.

Mit anderen Worten konnte die Oxidationsstabilität eines Farbstoffes vom allgemeinen Aufbau der Formel (I) durch Einführung von mindestens einem Substituenten an jedem der beiden Phenylringe des Farbstoffes erhöht werden. Aus diesem Grund kann keiner der beiden Reste R1 und R3 für ein Wasserstoffatom stehen.

Eine besonders gute Erhöhung der Oxidationsstabilität konnte bei Farbstoffen der allgemeinen Formel (I) beobachtet werden, bei welchen die Reste R1 und R3 unabhängig voneinander für eine C₁-C₆-Alkylgruppe oder für eine C₁-C₆-Alkoxygruppe stehen.

Ganz besonders bevorzugt ist es, wenn R1 und R3 unabhängig voneinander für eine C₁-C₆-Alkylgruppe stehen.

Weiterhin ist es ganz besonders bevorzugt, wenn R2 und R4 beide für ein Wasserstoffatom stehen.

In einer weiteren besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
- R1, R3: unabhängig voneinander für eine C₁-C₆-Alkylgruppeoder für eine C₁-C₆-Alkoxygruppe stehen,
- R2, R4: beide für ein Wasserstoffatom stehen.

Weiterhin tragen die direktziehenden Farbstoffe der allgemeinen Formel (I) die Reste R5 und R6. R5 und R6 können unabhängig voneinander für ein Wasserstoffatom, eine C₁-C₆-Alkylgruppe oder für eine C₂-C₆-Alkenylgruppe stehen. Besonders bevorzugt ist es, wenn R5 und R6 unabhängig voneinander für Wasserstoff oder für eine C₁-C₆-Alkylgruppe, insbesondere für eine Methylgruppe oder für eine Ethylgruppe, stehen. R5 und R6 können gleich oder verschieden sein, bevorzugt ist es, wenn R5 und R6 gleich sind.

In einer besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
- R5, R6: unabhängig voneinander für Wasserstoff oder für eine C₁-C₆-Alkylgruppe stehen.

Die heterozyklischen Endgruppen Het 1 und Het 2 der Formeln (II) bis (VII) tragen die Reste R7, R8, R9 und/oder R10.

Die Reste R7 und R10 sind jeweils an ein Stickstoffatom der Heterozyklen Het 1 und Het 2 gebunden. Hierbei können die Reste R7 und R10 jeweils unabhängig voneinander für eine C₁-C₆-Alkylgruppe oder für eine C₂-C₆-Alkenylgruppe stehen. Es ist bevorzugt, wenn R7 und R10 jeweils unabhängig voneinander für eine Methylgruppe oder eine Ethylgruppe stehen.

Die Reste R8 und R9 können jeweils unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen. Es ist bevorzugt, wenn R8 und R9 beide für ein Wasserstoffatom stehen.

In einer weiteren besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
- R7, R10: jeweils unabhängig voneinander für eine Methylgruppe oder eine Ethylgruppe stehen,
- R8, R9: für ein Wasserstoffatom stehen.

Bei der Gruppierung Q handelt es sich um eine strukturelle Einheit, welche die beiden einfach positiv geladenen Chromophore des Farbstoffs zum dikationischen Dimer verknüpft. Q steht für eine Gruppierung der Formel (VIII),

* -(CH₂)n-* (VIII)

- n: für eine ganze Zahl von 3 bis 6 steht,

Die beiden mit einem Stern markierten Positionen stellen hierbei jeweils die Verknüpfungspositionen zu den beiden N-Atomen der Formel (I) dar.

Überraschenderweise hat es sich für die Erzielung eines intensiven Farbergebnisses als grundsätzlich wichtig und erfindungswesentlich herausgestellt, dass die verbindende Gruppierung Q, welche die beiden einzelnen Chromophore miteinander verknüpft, eine Kettenlänge von mindestens 3 Atomen aufweist.

Aus diesem Grund steht n in der Formel (VIII) für eine ganze Zahl von mindestens 3. Die verbindende Gruppierung Q der Formel (VIII) umfasst demnach mindestens 3 C-Atome (d.h. hierbei handelt es sich um eine Gruppierung mit der Mindestlänge von -CH₂-CH₂-CH₂-).

Im Rahmen von Vergleichsversuchen hat sich herausgestellt, dass dimere Azofarbstoffe des prinzipiellen Typs der Formel (I), die jedoch eine nicht erfindungsgemäße Linker-Gruppe Q mit einer Länge von nur 2 C-Atomen besitzen, ein extrem schlechtes Farbaufzugsvermögen auf die Keratinfasern besitzen.

Während mit den erfindungsgemäßen Farbstoffen der Formel (I) intensive Färbungen erzielt werden konnten, führten Färbungen mit analogen dimeren Farbstoffen, die über eine kürzere Gruppierung Q mit einer Kettenlänge von nur 2 C-Atomen verknüpft sind, zu praktisch überhaupt keinem Farbaufzug auf den keratinischen Fasern.

Ohne auf eine Theorie beschränkt zu sein, könnte möglicherweise eine mit der kurzen Linker-Kette Q verbundene starre Geometrie und hierdurch bedingt eine ungünstige räumliche Konformation des Farbstoffes die Diffusion der kurzkettigen dimeren Farbstoffe in die keratinische Faser hinein verschlechtern.

In einer ganz besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
- Q: für eine Gruppierung der Formel (VIII) steht, *-(CH₂)n-* (VIII)
- und n: für eine ganze Zahl von 3 bis 6 steht.

In einer explizit ganz besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
- Q: für eine Gruppierung der Formel (VIII) steht, *-(CH₂)n-* (VIII)
- und n: für die Zahl 3 steht.

Bei den erfindungsgemäßen Farbstoffen der Formel (I) handelt es sich um dimere Azofarbstoffe, die zweifach positiv geladen sind. Die beiden positiven Ladungen werden durch die anionischen Gegenionen X neutralisiert. Hierbei ist der dikationische organische Teil für die Färbung der Keratinfasern verantwortlich. Die Gegenionen X dienen lediglich der Wahrung der Elektroneutralität, so dass die genaue Natur der Gegenionen X für die Erzielung des gewünschten Farbergebnisses keine wesentliche Rolle spielt. Da der Farbstoff in einem kosmetischen Mittel eingesetzt wird, müssen die Gegenionen X physiologisch verträglich sein. Physiologisch verträglich bedeutet in diesem Zusammenhang zum Einsatz im kosmetischen Mittel (d.h. zur Anwendung auf dem menschlichen Haar und der menschlichen Haut) geeignet. Bei X handelt es sich um physiologisch verträgliche Anionen, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat.

Unter Chlorid wird ein Anion Cl⁻ verstanden. Unter Bromid wird ein Anion Br⁻ verstanden. Unter lodid wird ein Anion J⁻ verstanden. Unter Methylsulfat wird ein Anion H₃COSO₄⁻ verstanden.

Unter Methylsulfonat wird ein Anion H₃CSO₃⁻ verstanden. Unter p-Toluolsulfonat wird ein Anion H₃C(C₆H₄)SO₃⁻ verstanden. Unter Acetat wird ein Anion H₃CCOO⁻ verstanden. Unter Hydrogensulfat wird ein Anion HSO₄⁻ verstanden.

Unter ½ Sulfat wird ein halbes Äquivalent des doppelt negativ geladenen Anions SO₄²⁻ verstanden. Unter ½ Tetrachlorozinkat wird ein halbes Äquivalent des doppelt negativ geladenen Anions ZnCl₄²⁻verstanden. Bei Sulfat und Tetrachlorozinkat ist es demzufolge ebenfalls möglich und auch erfindungsgemäß, wenn der dikationische Farbstoff der Formel (I) durch ein SO₄²⁻ Ion bzw. durch ein ZnCl₄²⁻ Ion neutralisiert wird.

Zusammenfassend sind ganz besonders bevorzugte Mittel zur Farbveränderung von keratinischen Fasern dadurch gekennzeichnet, dass sie in einem kosmetischen Träger (a) mindestens einen direktziehenden Farbstoff der Formel (la) enthalten, 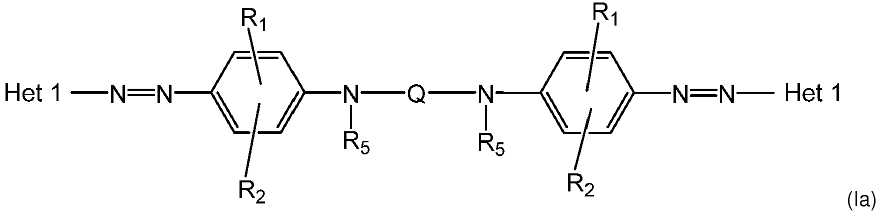 wobei
- Het 1: für eine der Strukturen (II), (III) oder (IV) steht,

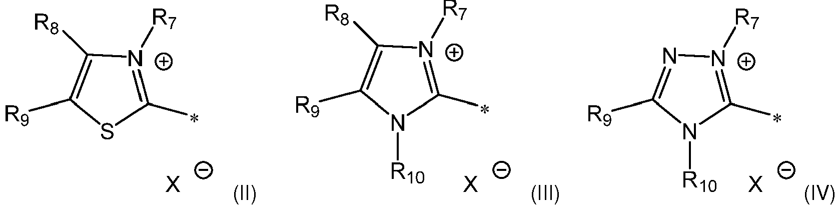

- R1: für eine C₁-C₆-Alkylgruppe für eine C₁-C₆-Alkoxygruppe steht,
- R2: für Wasserstoff steht
- R5: für Wasserstoff oder für eine C₁-C₆-Alkylgruppesteht
- Q: für eine Gruppierung der Formel (VIII) steht,

*-(CH₂)n-* (VIII)
- n: für eine ganze Zahl von 3 bis 6 steht,
- R7, R10: unabhängig voneinander für eine C₁-C₆-Alkylgruppe stehen,
- R8, R9: für Wasserstoff stehen,
- X-: für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat steht.

In einer weiteren bevorzugten Ausführungsform ist ein Mittel zum Färben von keratinischen Fasern dadurch gekennzeichnet, dass es mindestens eine Verbindung der allgemeinen Formel (I) enthält, die ausgewählt ist aus
- Salzen des 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-iums 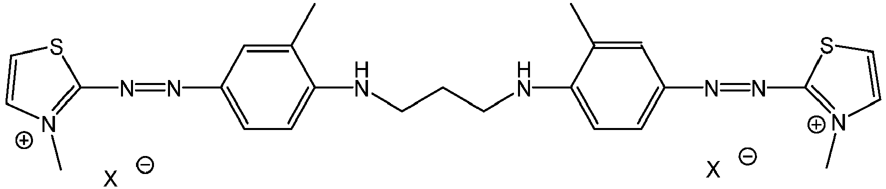
- Salzen des 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-iums 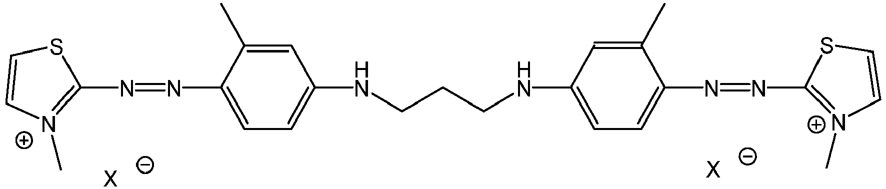
- Salzen des 3-Methyl-2-[2-(3-methyl-4-{[4-({2-methyl-4-[2-(3-methyl-1,3-thiazol-3-ium-2-yl)diazen-1-yl]phenyl}amino)butyl]amino}phenyl)diazen-1-yl]-1 ,3-thiazol-3-iums 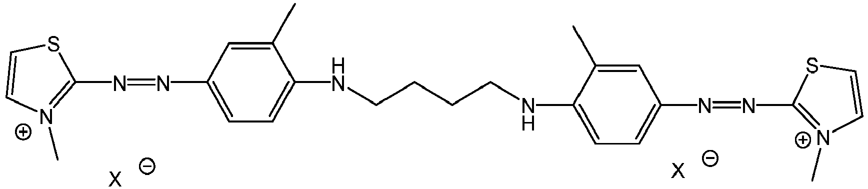
- Salzen des 3-Methyl-2-[2-(2-methyl-4-{[4-({3-methyl-4-[2-(3-methyl-1,3-thiazol-3-ium-2-yl)diazen-1-yl]phenyl}amino)butyl]amino}phenyl)diazen-1-yl]-1 ,3-thiazol-3-iums 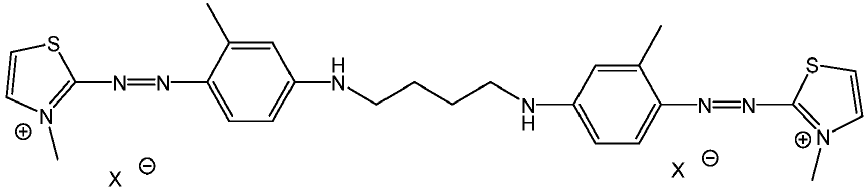
- Salzen des 3-Methyl-2-[2-(3-methyl-4-{[5-({2-methyl-4-[2-(3-methyl-1,3-thiazol-3-ium-2-yl)diazen-1-yl]phenyl}amino)pentyl]amino}phenyl)diazen-1-yl]-1,3-thiazol-3-iums 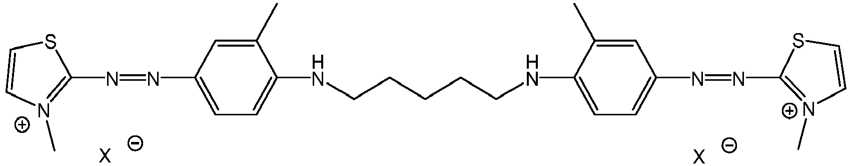
- Salzen des 3-Methyl-2-[2-(2-methyl-4-{[5-({3-methyl-4-[2-(3-methyl-1,3-thiazol-3-ium-2-yl)diazen-1-yl]phenyl}amino)pentyl]amino}phenyl)diazen-1-yl]-1,3-thiazol-3-iums 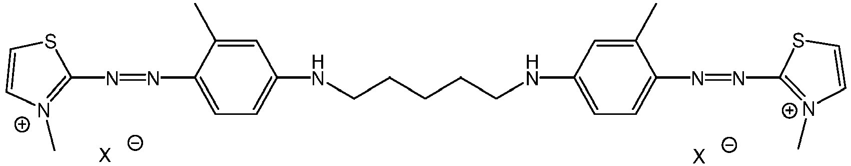
- Salzen des 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-iums 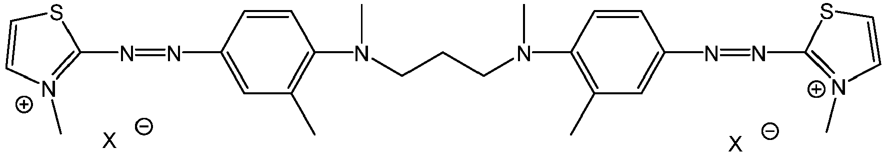
- Salzen des 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-iums 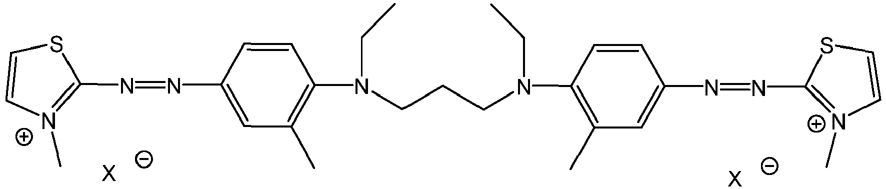
- Salzen des 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-iums 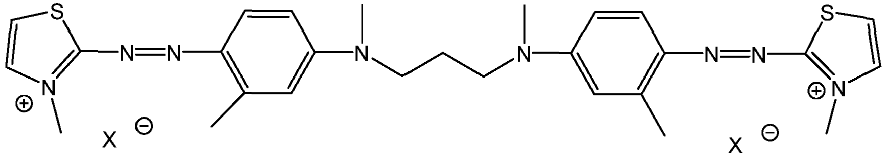
- Salzen des 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-iums 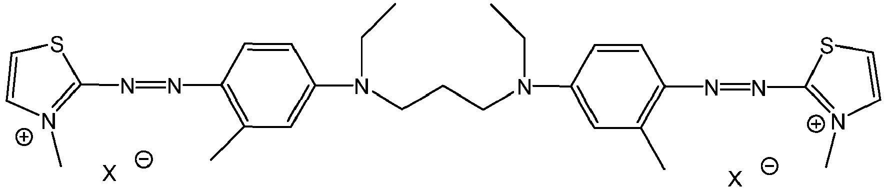
- Salzen des 3-Methyl-2-(2-{3-methyl-4-[methyl({4-[methyl({2-methyl-4-[2-(3-methyl-1,3-thiazol-3-ium-2-yl)diazen-1-yl]phenyl})amino]butyl})amino]phenyl}diazen-1-yl)-1,3-thiazol-3-iums 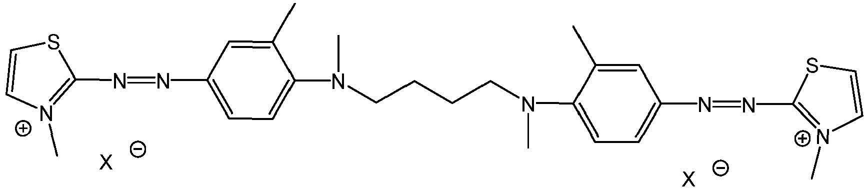
- Salzen des 3-Methyl-2-(2-{2-methyl-4-[methyl({4-[methyl({3-methyl-4-[2-(3-methyl-1,3-thiazol-3-ium-2-yl)diazen-1-yl]phenyl})amino]butyl})amino]phenyl}diazen-1-yl)-1,3-thiazol-3-iums 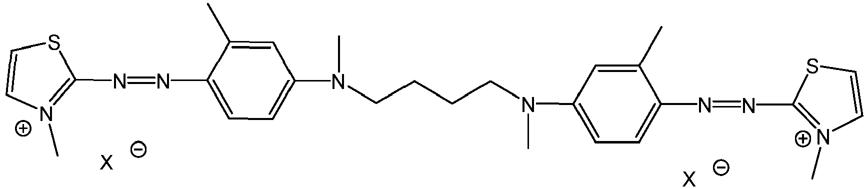
- Salzen des 3-Methyl-2-(2-{3-methyl-4-[methyl({5-[methyl({2-methyl-4-[2-(3-methyl-1,3-thiazol-3-ium-2-yl)diazen-1-yl]phenyl})amino]pentyl})amino]phenyl}diazen-1-yl)-1,3-thiazol-3-iums 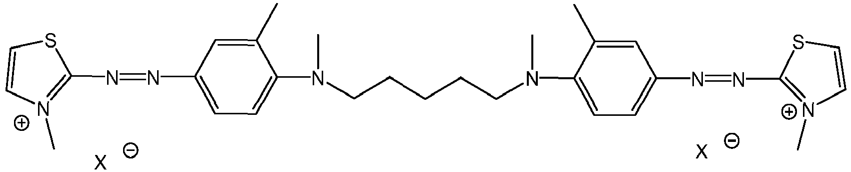
- Salzen des 3-Methyl-2-(2-{2-methyl-4-[methyl({5-[methyl({3-methyl-4-[2-(3-methyl-1,3-thiazol-3-ium-2-yl)diazen-1-yl]phenyl})amino]pentyl})amino]phenyl}diazen-1-yl)-1,3-thiazol-3-iums 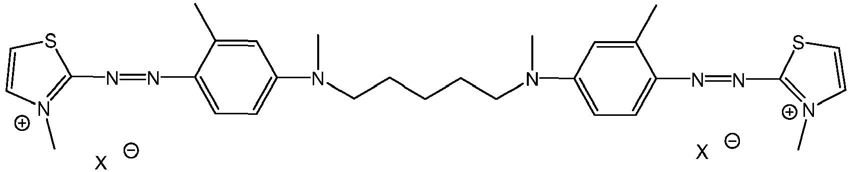
- Salzen des 2-[2-(3-Methoxy-4-{[3-({2-methoxy-4-[2-(3-methyl-1,3-thiazol-3-ium-2-yl)diazen-1-yl]phenyl}amino)propyl]amino}phenyl)diazen-1-yl]-3-methyl-1,3-thiazol-3-iums 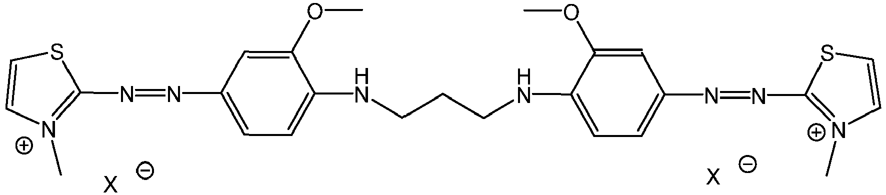
- Salzen des 2-[2-(2-Methoxy-4-{[3-({3-methoxy-4-[2-(3-methyl-1,3-thiazol-3-ium-2-yl)diazen-1-yl]phenyl}amino)propyl]amino}phenyl)diazen-1-yl]-3-methyl-1,3-thiazol-3-iums 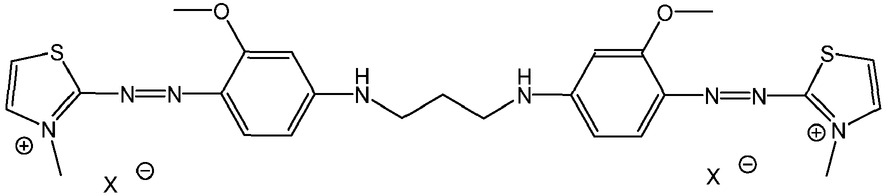
- Salzen des 2-[2-(3-Methoxy-4-{[4-({2-methoxy-4-[2-(3-methyl-1,3-thiazol-3-ium-2-yl)diazen-1-yl]phenyl}amino)butyl]amino}phenyl)diazen-1-yl]-3-methyl-1,3-thiazol-3-iums 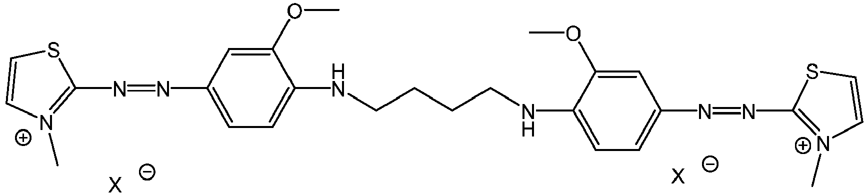
- Salzen des 2-[2-(2-Methoxy-4-{[4-({3-methoxy-4-[2-(3-methyl-1,3-thiazol-3-ium-2-yl)diazen-1-yl]phenyl}amino)butyl]amino}phenyl)diazen-1-yl]-3-methyl-1,3-thiazol-3-iums 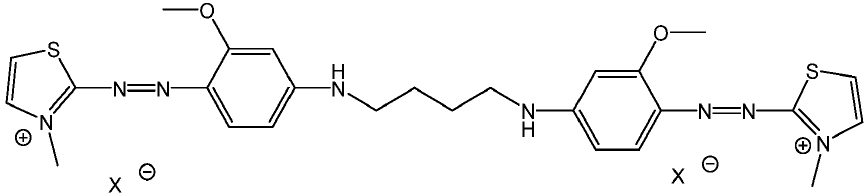
- Salzen des 2-[2-(3-Methoxy-4-{[5-({2-methoxy-4-[2-(3-methyl-1,3-thiazol-3-ium-2-yl)diazen-1-yl]phenyl}amino)pentyl]amino}phenyl)diazen-1-yl]-3-methyl-1,3-thiazol-3-iums 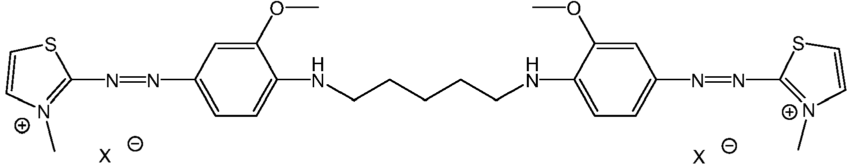
- Salzen des 2-[2-(2-Methoxy-4-{[5-({3-methoxy-4-[2-(3-methyl-1,3-thiazol-3-ium-2-yl)diazen-1-yl]phenyl}amino)pentyl]amino}phenyl)diazen-1-yl]-3-methyl-1,3-thiazol-3-iums 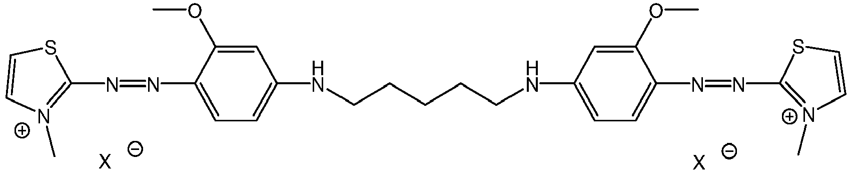
- Salzen des 2-[2-(3-Methoxy-4-{[3-({2-methoxy-4-[2-(3-methyl-1,3-thiazol-3-ium-2-yl)diazen-1-yl]phenyl}(methyl)amino)propyl](methyl)amino}phenyl)diazen-1-yl]-3-methyl-1,3-thiazol-3-iums 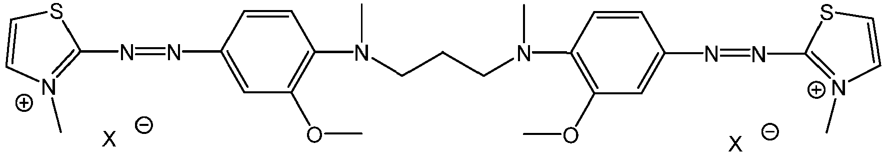
- Salzen des 2-[2-(2-Methoxy-4-{[3-({3-methoxy-4-[2-(3-methyl-1,3-thiazol-3-ium-2-yl)diazen-1-yl]phenyl}(methyl)amino)propyl](methyl)amino}phenyl)diazen-1-yl]-3-methyl-1,3-thiazol-3-iums 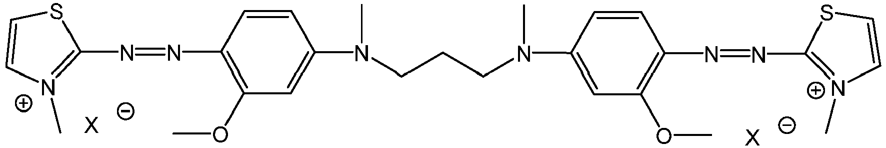
- Salzen des 2-[2-(3-Methoxy-4-{[4-({2-methoxy-4-[2-(3-methyl-1,3-thiazol-3-ium-2-yl)diazen-1-yl]phenyl}(methyl)amino)butyl](methyl)amino}phenyl)diazen-1-yl]-3-methyl-1,3-thiazol-3-iums 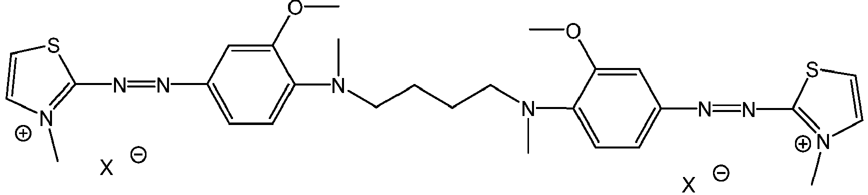
- Salzen des 2-[2-(2-Methoxy-4-{[4-({3-methoxy-4-[2-(3-methyl-1,3-thiazol-3-ium-2-yl)diazen-1-yl]phenyl}(methyl)amino)butyl](methyl)amino}phenyl)diazen-1-yl]-3-methyl-1,3-thiazol-3-iums 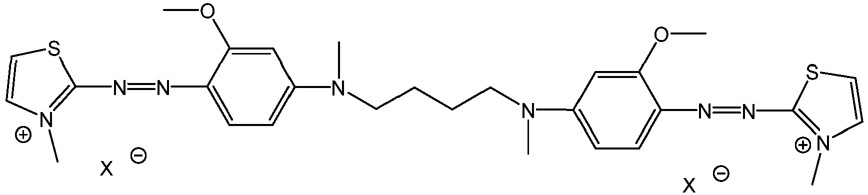
- Salzen des 2-[2-(3-Methoxy-4-{[5-({2-methoxy-4-[2-(3-methyl-1,3-thiazol-3-ium-2-yl)diazen-1-yl]phenyl}(methyl)amino)pentyl](methyl)amino}phenyl)diazen-1-yl]-3-methyl-1,3-thiazol-3-iums 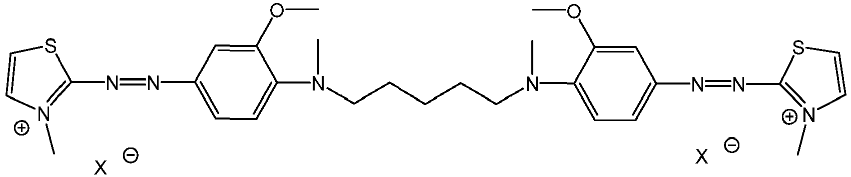
- Salzen des 2-[2-(2-Methoxy-4-{[5-({3-methoxy-4-[2-(3-methyl-1,3-thiazol-3-ium-2-yl)diazen-1-yl]phenyl}(methyl)amino)pentyl](methyl)amino}phenyl)diazen-1-yl]-3-methyl-1,3-thiazol-3-iums 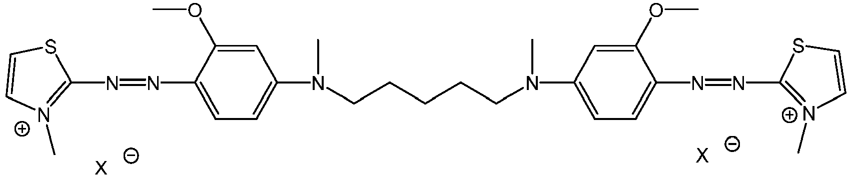
- Salzen des 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-iums 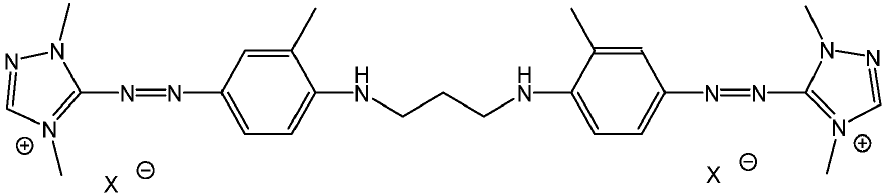
- Salzen des 5-[2-(4-{[3-({4-[2-(1,4-Dimethyl-1 H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-iums 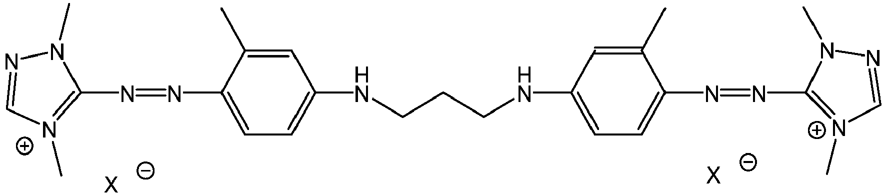
- Salzen des 5-[2-(4-{[4-({4-[2-(1,4-Dimethyl-1 H-1,2,4-triazol-4-ium-5-yl)diazen-1-yl]-2-methylphenyl}amino)butyl]amino}-3-methylphenyl)diazen-1-yl]-1,4-dimethyl-1H-1,2,4-triazol-4-iums 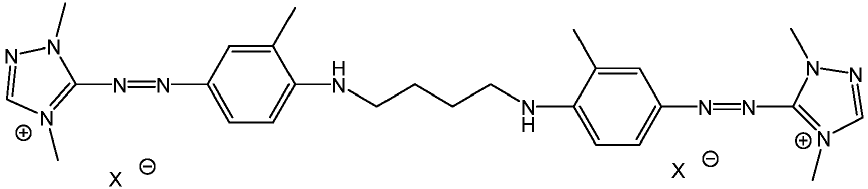
- Salzen des 5-[2-(4-{[4-({4-[2-(1,4-Dimethyl-1 H-1,2,4-triazol-4-ium-5-yl)diazen-1-yl]-3-methylphenyl}amino)butyl]amino}-2-methylphenyl)diazen-1-yl]-1,4-dimethyl-1H-1,2,4-triazol-4-iums 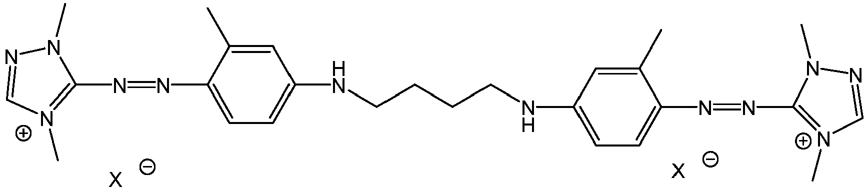
- Salzen des 5-[2-(4-{[5-({4-[2-(1,4-Dimethyl-1H-1,2,4-triazol-4-ium-5-yl)diazen-1-yl]-2-methylphenyl}amino)pentyl]amino}-3-methylphenyl)diazen-1-yl]-1,4-dimethyl-1H-1,2,4-triazol-4-iums 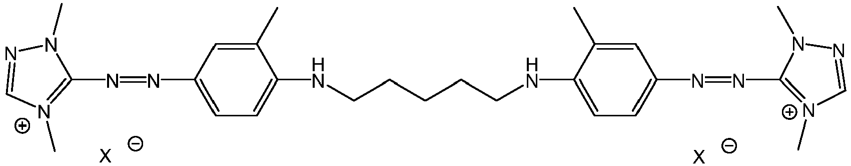
- Salzen des 5-[2-(4-{[5-({4-[2-(1,4-Dimethyl-1H-1,2,4-triazol-4-ium-5-yl)diazen-1-yl]-3-methylphenyl}amino)pentyl]amino}-2-methylphenyl)diazen-1-yl]-1,4-dimethyl-1H-1,2,4-triazol-4-iums 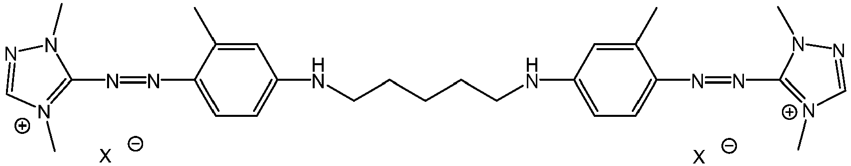
- Salzen des 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-iums 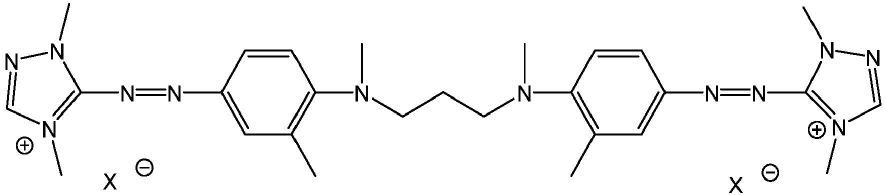
- Salzen des 5-[2-(4-{[3-({4-[2-(1,4-Dimethyl-1H-1,2,4-triazol-4-ium-5-yl)diazen-1-yl]-3-methylphenyl}(methyl)amino)propyl](methyl)aminol-2-methylphenyl)diazen-1-yl]-1,4-dimethyl-1H-1,2,4-triazol-4-iums 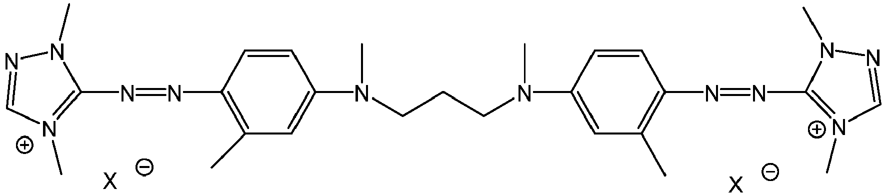
- Salzen des 5-[2-(4-{[4-({4-[2-(1,4-Dimethyl-1H-1,2,4-triazol-4-ium-5-yl)diazen-1-yl]-2-methylphenyl}(methyl)amino)butyl](methyl)amino}-3-methylphenyl)diazen-1-yl]-1,4-dimethyl-1H-1,2,4-triazol-4-iums 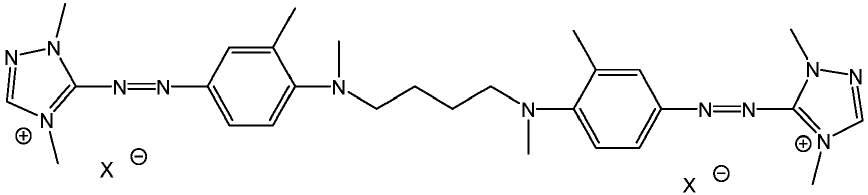
- Salzen des 5-[2-(4-{[4-({4-[2-(1,4-Dimethyl-1H-1,2,4-triazol-4-ium-5-yl)diazen-1-yl]-3-methylphenyl}(methyl)amino)butyl](methyl)amino}-2-methylphenyl)diazen-1-yl]-1,4-dimethyl-1H-1,2,4-triazol-4-iums 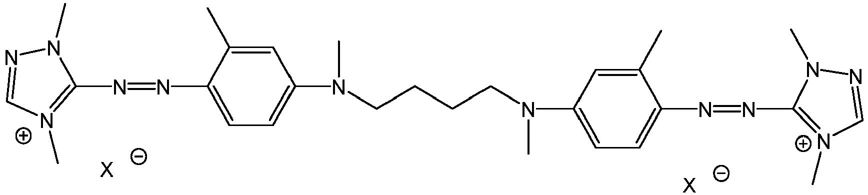
- Salzen des 5-[2-(4-{[5-({4-[2-(1,4-Dimethyl-1H-1,2,4-triazol-4-ium-5-yl)diazen-1-yl]-2-methylphenyl}(methyl)amino)pentyl](methyl)amino}-3-methylphenyl)diazen-1-yl]-1,4-dimethyl-1H-1,2,4-triazol-4-iums 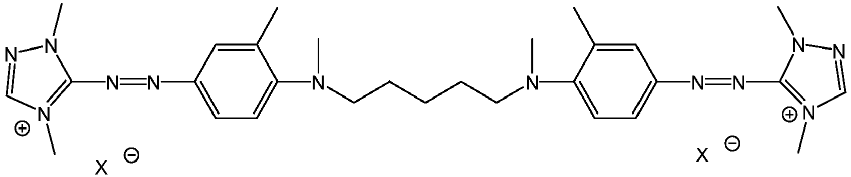
- Salzen des 5-[2-(4-{[5-({4-[2-(1,4-Dimethyl-1H-1,2,4-triazol-4-ium-5-yl)diazen-1-yl]-3-methylphenyl}(methyl)amino)pentyl](methyl)amino}-2-methylphenyl)diazen-1-yl]-1,4-dimethyl-1H-1,2,4-triazol-4-iums 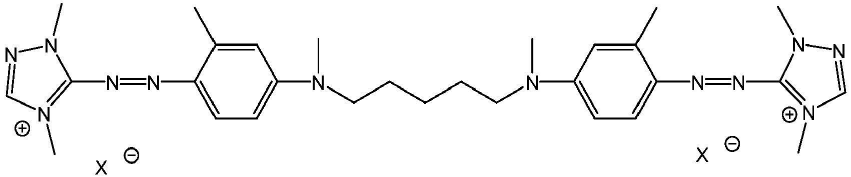
- Salzen des 5-[2-(4-{[3-({4-[2-(1,4-Dimethyl-1H-1,2,4-triazol-4-ium-5-yl)diazen-1-yl]-2-methoxyphenyl}amino)propyl]amino}-3-methoxyphenyl)diazen-1-yl]-1,4-dimethyl-1H-1,2,4-triazol-4-iums 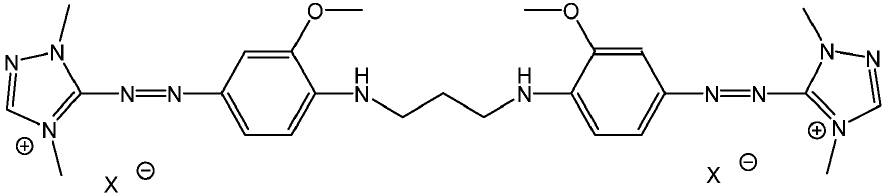
- Salzen des 5-[2-(4-{[3-({4-[2-(1,4-Dimethyl-1H-1,2,4-triazol-4-ium-5-yl)diazen-1-yl]-3-methoxyphenyl}amino)propyl]amino}-2-methoxyphenyl)diazen-1-yl]-1,4-dimethyl-1H-1,2,4-triazol-4-iums 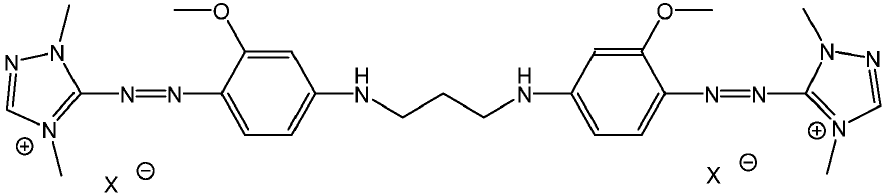
- Salzen des 5-[2-(4-{[4-({4-[2-(1,4-Dimethyl-1H-1,2,4-triazol-4-ium-5-yl)diazen-1-yl]-2-methoxyphenyl}amino)butyl]amino}-3-methoxyphenyl)diazen-1-yl]-1,4-dimethyl-1H-1 ,2,4-triazol-4-iums 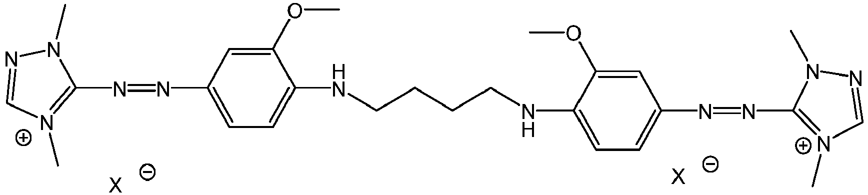
- Salzen des 5-[2-(4-{[4-({4-[2-(1,4-Dimethyl-1H-1,2,4-triazol-4-ium-5-yl)diazen-1-yl]-3-methoxyphenyl}amino)butyl]amino}-2-methoxyphenyl)diazen-1-yl]-1,4-dimethyl-1H-1,2,4-triazol-4-iums 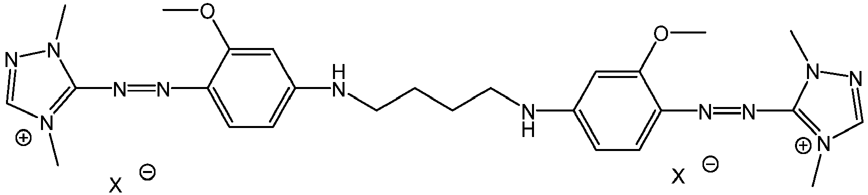
- Salzen des 5-[2-(4-{[5-({4-[2-(1,4-Dimethyl-1 H-1,2,4-triazol-4-ium-5-yl)diazen-1-yl]-2-methoxyphenyl}amino)pentyl]amino}-3-methoxyphenyl)diazen-1-yl]-1,4-dimethyl-1H-1,2,4-triazol-4-iums 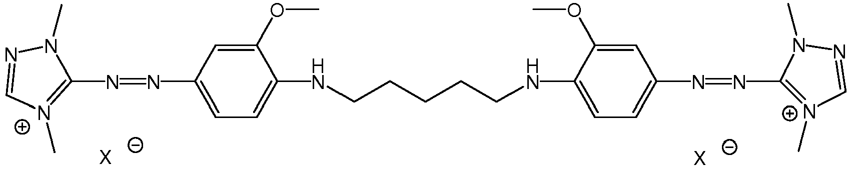
- Salzen des 5-[2-(4-{[5-({4-[2-(1,4-Dimethyl-1 H-1,2,4-triazol-4-ium-5-yl)diazen-1-yl]-3-methoxyphenyl}amino)pentyl]amino}-2-methoxyphenyl)diazen-1-yl]-1,4-dimethyl-1H-1,2,4-triazol-4-iums 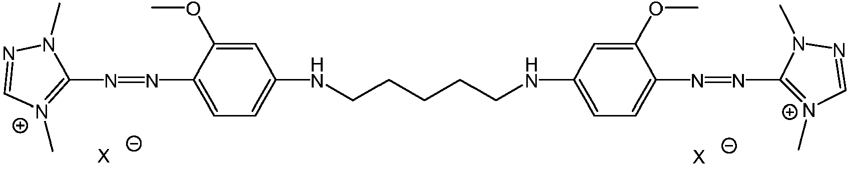
- Salzen des 5-[2-(4-{[3-({4-[2-(1,4-Dimethyl-1H-1,2,4-triazol-4-ium-5-yl)diazen-1-yl]-2-methoxyphenyl}(methyl)amino)propyl](methyl)amino}-3-methoxyphenyl)diazen-1-yl]-1,4-dimethyl-1H-1,2,4-triazol-4-iums 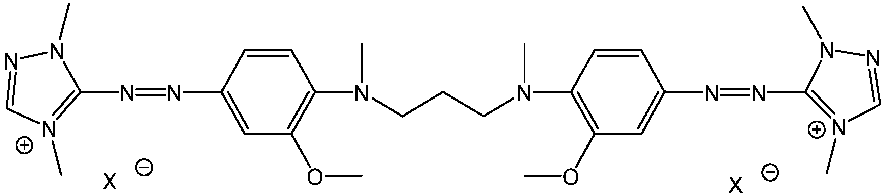
- Salzen des 5-[2-(4-{[3-({4-[2-(1,4-Dimethyl-1H-1,2,4-triazol-4-ium-5-yl)diazen-1-yl]-3-methoxyphenyl}(methyl)amino)propyl](methyl)amino}-2-methoxyphenyl)diazen-1-yl]-1,4-dimethyl-1H-1,2,4-triazol-4-iums 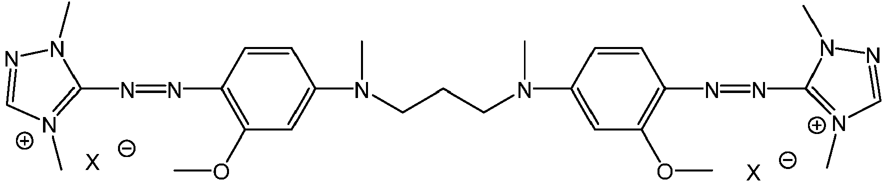
- Salzen des 5-[2-(4-{[4-({4-[2-(1,4-Dimethyl-1H-1,2,4-triazol-4-ium-5-yl)diazen-1-yl]-2-methoxyphenyl}(methyl)amino)butyl](methyl)amino}-3-methoxyphenyl)diazen-1-yl]-1,4-dimethyl-1H-1,2,4-triazol-4-iums 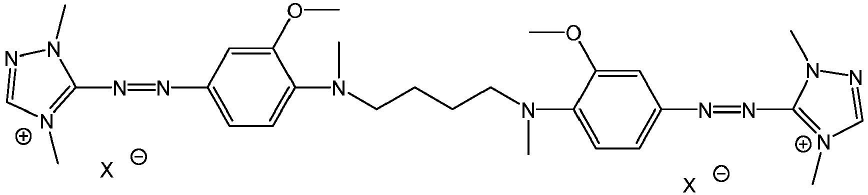
- Salzen des 5-[2-(4-{[4-({4-[2-(1,4-Dimethyl-1H-1,2,4-triazol-4-ium-5-yl)diazen-1-yl]-3-methoxyphenyl}(methyl)amino)butyl](methyl)amino}-2-methoxyphenyl)diazen-1-yl]-1,4-dimethyl-1H-1,2,4-triazol-4-iums 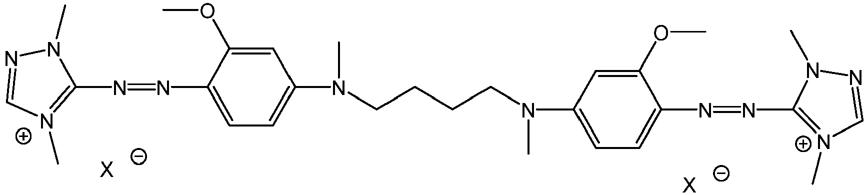
- Salzen des 5-[2-(4-{[5-({4-[2-(1,4-Dimethyl-1H-1,2,4-triazol-4-ium-5-yl)diazen-1-yl]-2-methoxyphenyl}(methyl)amino)pentyl](methyl)amino}-3-methoxyphenyl)diazen-1-yl]-1,4-dimethyl-1H-1,2,4-triazol-4-iums 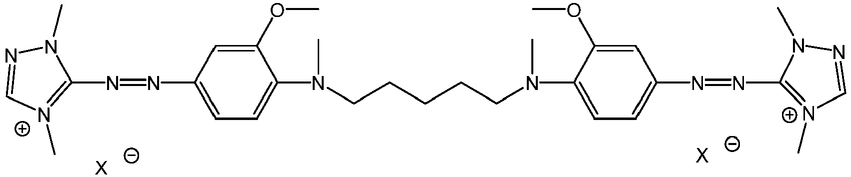
- Salzen des 5-[2-(4-{[5-({4-[2-(1,4-Dimethyl-1H-1,2,4-triazol-4-ium-5-yl)diazen-1-yl]-3-methoxyphenyl}(methyl)amino)pentyl](methyl)amino}-2-methoxyphenyl)diazen-1-yl]-1,4-dimethyl-1H-1,2,4-triazol-4-iums

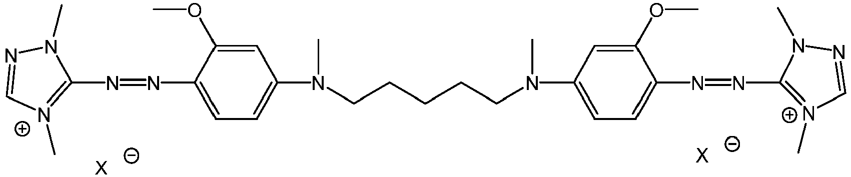

Bei den vorgenannten Verbindungen handelt es sich um dikationische dimere Farbstoffe, wobei das organische Dikation durch zwei Anionen X- neutralisiert wird. Bei den Anionen X- handelt es sich um ein physiologisch verträgliches Anion, bevorzugt aus der Gruppe aus Chlorid, Bromid, lodid, Methylsulfat, Methylsulfonat, p-Toluolsulfonat, Acetat, Hydrogensulfat, ½ Sulfat oder ½ Tetrachlorozinkat.

Im Rahmen der zu dieser Erfindung führenden Arbeiten hat sich herausgestellt, dass mit den direktziehenden Farbstoffen (a) der Formel (I) besonders dann ganz besonders intensive und oxidationsstabile Färbungen erzielt werden konnten, wenn
- Het1, Het2: unabhängig voneinander für eine der Strukturen (II), (III) oder (IV) stehen.

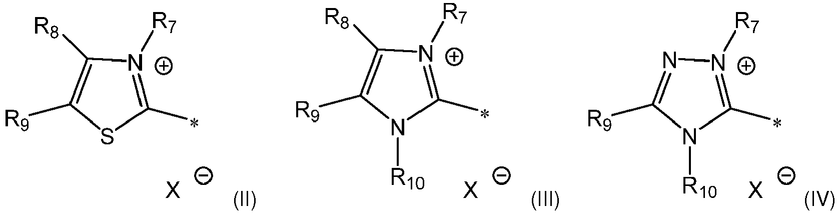

- R1, R3: beide für eine Methylgruppe stehen
- R2, R4: beide für ein Wasserstoffatom stehen.
- R5, R6: unabhängig voneinander für Wasserstoff oder für eine C₁-C₆-Alkylgruppe stehen.
- R7, R10: beide für eine Methylgruppe stehen
- R8, R9: beide für ein Wasserstoffatom stehen.
- Q: für eine Gruppierung der Formel (VIII) steht,

*-(CH₂)n-* (VIII)
- und n: für die Zahl 3 oder 4 steht.

Explizit ganz besonders bevorzugt sind daher erfindungsgemäße Mittel zum Färben von keratinischen Fasern, welche mindestens einen Farbstoff (a) der Formel (I) enthalten, der ausgewählt ist aus der Gruppe aus
- Salzen des 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-iums 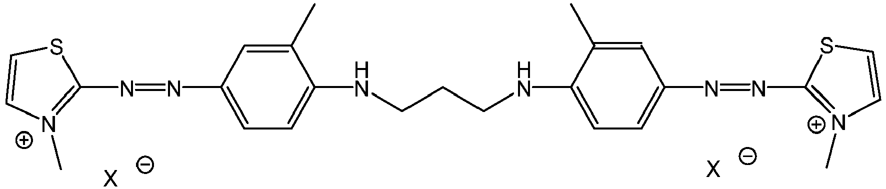
- Salzen des 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-iums 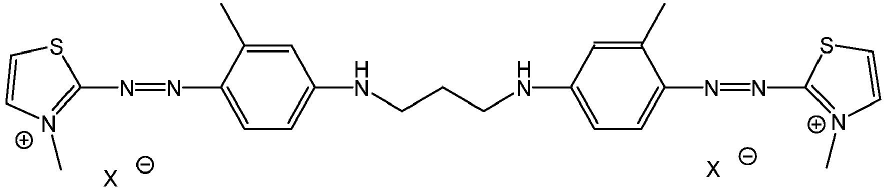
- Salzen des 3-Methyl-2-[2-(3-methyl-4-{[4-({2-methyl-4-[2-(3-methyl-1,3-thiazol-3-ium-2-yl)diazen-1-yl]phenyl}amino)butyl]amino}phenyl)diazen-1-yl]-1 ,3-thiazol-3-iums 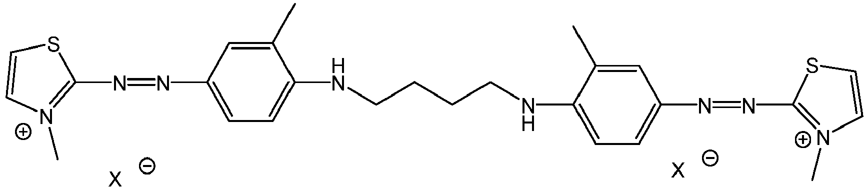
- Salzen des 3-Methyl-2-[2-(2-methyl-4-{[4-({3-methyl-4-[2-(3-methyl-1,3-thiazol-3-ium-2-yl)diazen-1-yl]phenyl}amino)butyl]amino}phenyl)diazen-1-yl]-1 ,3-thiazol-3-iums 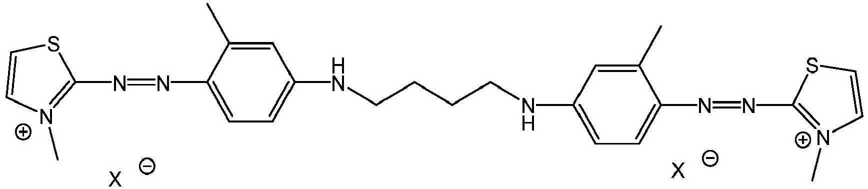
- Salzen des 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-iums 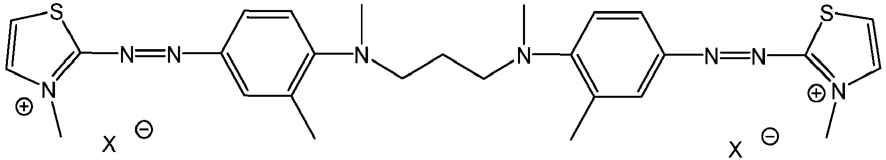
- Salzen des 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-iums 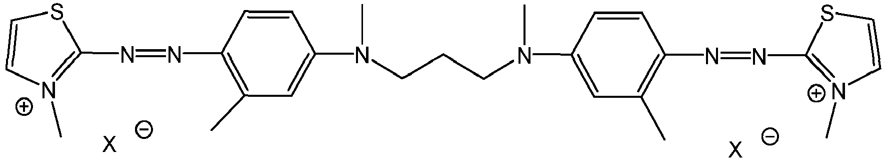
- Salzen des 3-Methyl-2-(2-{3-methyl-4-[methyl({4-[methyl({2-methyl-4-[2-(3-methyl-1,3-thiazol-3-ium-2-yl)diazen-1-yl]phenyl})amino]butyl})amino]phenyl}diazen-1-yl)-1,3-thiazol-3-iums 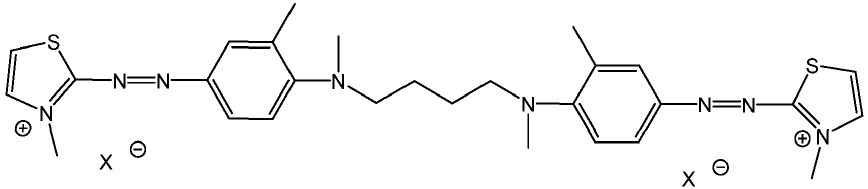
- Salzen des 3-Methyl-2-(2-{2-methyl-4-[methyl({4-[methyl({3-methyl-4-[2-(3-methyl-1,3-thiazol-3-ium-2-yl)diazen-1-yl]phenyl})amino]butyl})amino]phenyl}diazen-1-yl)-1,3-thiazol-3-iums 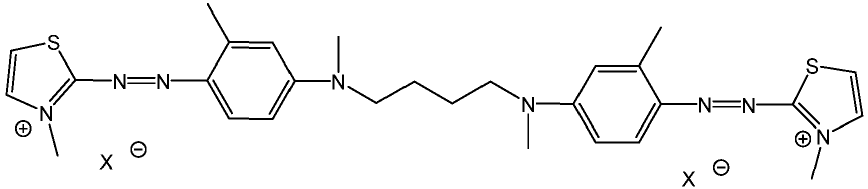
- Salzen des 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-iums 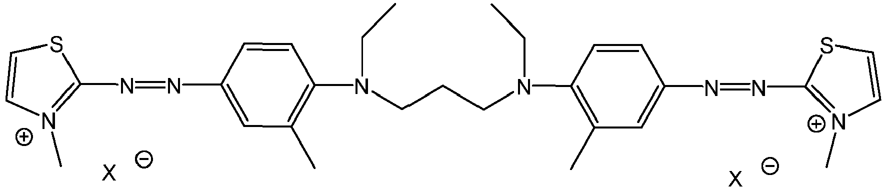
- Salzen des 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-iums 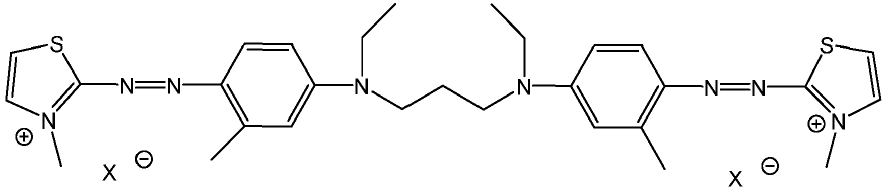
- Salzen des 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-iums 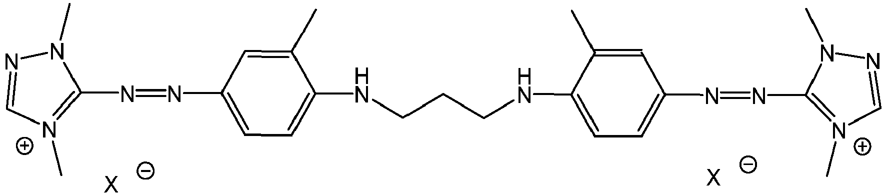
- Salzen des 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-iums 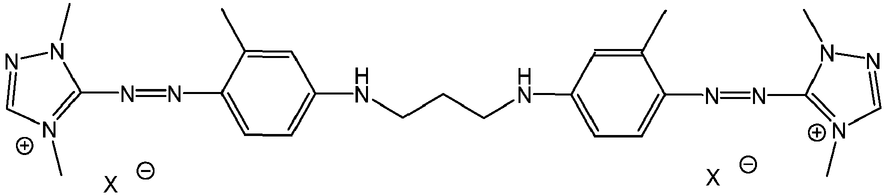
- Salzen des 5-[2-(4-{[4-({4-[2-(1,4-Dimethyl-1H-1,2,4-triazol-4-ium-5-yl)diazen-1-yl]-2-methylphenyl}amino)butyl]amino}-3-methylphenyl)diazen-1-yl]-1,4-dimethyl-1H-1,2,4-triazol-4-iums 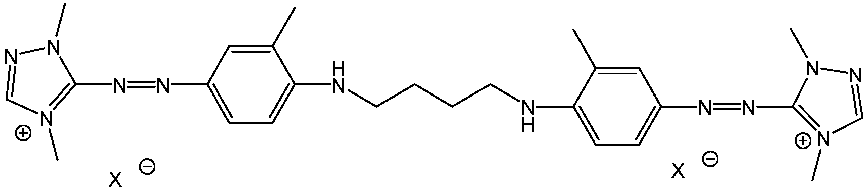
- Salzen des 5-[2-(4-{[4-({4-[2-(1,4-Dimethyl-1H-1,2,4-triazol-4-ium-5-yl)diazen-1-yl]-3-methylphenyl}amino)butyl]amino}-2-methylphenyl)diazen-1-yl]-1,4-dimethyl-1H-1,2,4-triazol-4-iums 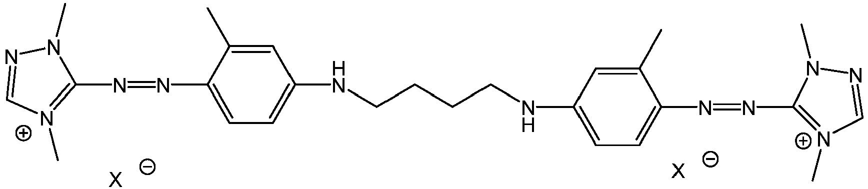
- Salzen des 5-[2-(4-{[3-({4-[2-(1,4-Dimethyl-1H-1,2,4-triazol-4-ium-5-yl)diazen-1-yl]-2-methylphenyl}(methyl)amino)propyl](methyl)aminol-3-methylphenyl)diazen-1-yl]-1,4-dimethyl-1H-1,2,4-triazol-4-iums 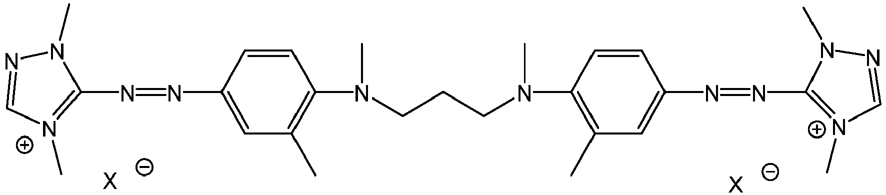
- Salzen des 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-iums 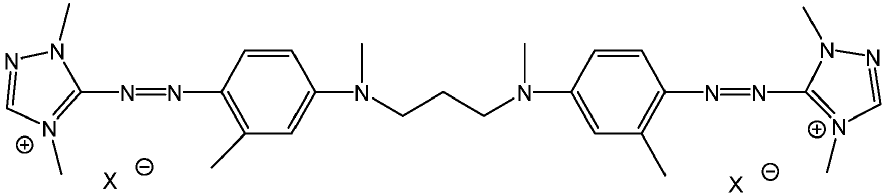
- Salzen des 5-[2-(4-{[4-({4-[2-(1,4-Dimethyl-1 H-1,2,4-triazol-4-ium-5-yl)diazen-1-yl]-2-methylphenyl}(methyl)amino)butyl](methyl)amino}-3-methylphenyl)diazen-1-yl]-1,4-dimethyl-1H-1,2,4-triazol-4-iums 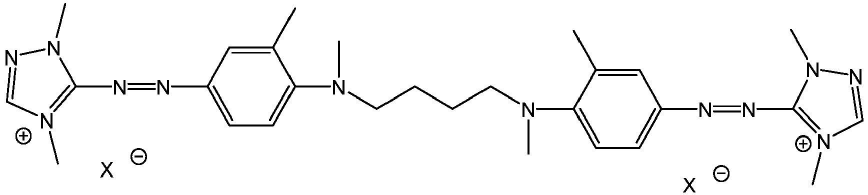
- Salzen des 5-[2-(4-{[4-({4-[2-(1,4-Dimethyl-1H-1,2,4-triazol-4-ium-5-yl)diazen-1-yl]-3-methylphenyl}(methyl)amino)butyl](methyl)aminol-2-methylphenyl)diazen-1-yl]-1,4-dimethyl-1H-1,2,4-triazol-4-iums

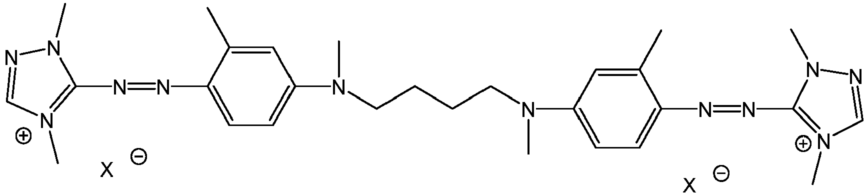

In einer bevorzugten Ausführungsform ist ein erfindungsgemäßes Mittel zum Färben von keratinischen Fasern dadurch gekennzeichnet, dass es als Farbstoff der Formel (a) mindestens eine Verbindung enthält, die ausgewählt ist aus der Gruppe aus
- 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium Dichlorid
- 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium Dibromid
- 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium Sulfat
- 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium Di(toluolsulfonat)
- 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium Di(methylsulfat)
- 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium Tetrachlorozinkat
- 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium Dichlorid
- 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium Dibromid
- 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium Sulfat
- 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium Di(toluolsulfonat)
- 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium Di(methylsulfat)
- 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium Tetrachlorozinkat
- 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dichlorid
- 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dibromid
- 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Sulfat
- 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Tetrachlorozinkat
- 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dichlorid
- 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dibromid
- 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Sulfat
- 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Tetrachlorozinkat
- 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium Dichlorid
- 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium Dibromid
- 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium Sulfat
- 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium Di(toluolsulfonat)
- 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium Di(methylsulfat)
- 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium Tetrachlorozinkat
- 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium Dichlorid
- 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium Dibromid
- 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium Sulfat
- 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium Di(toluolsulfonat)
- 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium Di(methylsulfat)
- 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium Tetrachlorozinkat
- 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium Dichlorid
- 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium Dibromid
- 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium Sulfat
- 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium Di(toluosulfonat)
- 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium Di(methylsulfat)
- 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium Tetrachlorozinkat
- 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium Dichlorid
- 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium Dibromid
- 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium Sulfat
- 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium Di(toluolsulfonat)
- 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium Di(methylsulfat)
- 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium Tetrachlorozinkat

In einer explizit ganz besonder bevorzugten Ausführungsform ist ein erfindungsgemäßes Mittel zum Färben von keratinischen Fasern dadurch gekennzeichnet, dass es als Farbstoff der Formel (a) mindestens eine Verbindung enthält, die ausgewählt ist aus der Gruppe aus
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium 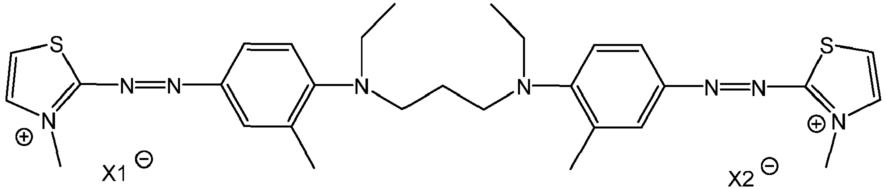
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dichlorid
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dibromid
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Sulfat
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Tetrachlorozinkat 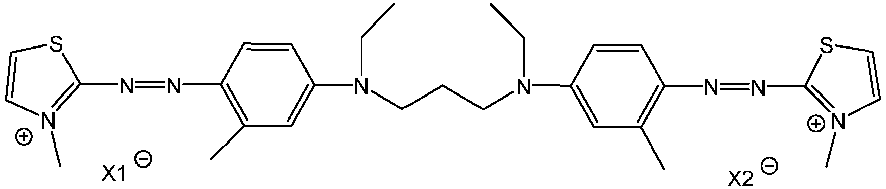
- 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dichlorid
- 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Dibromid
- 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Sulfat
- 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium Tetrachlorozinkat

Die erfindungsgemäßen Mittel zur Farbveränderung von keratinischen Fasern enthalten den oder die direktziehenden Farbstoffe der Formel (I) vorzugsweise in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-%. Die Mengenangabe in Gewichtsprozent bezieht sich hierbei auf die Gesamtmenge aller im Mittel enthaltenen Verbindungen der Formel (I), die zum Gesamtgewicht des Mittels in Relation gesetzt wird.

In einer weiteren bevorzugten Ausführungsform ist ein erfindungsgemäßes Mittel zum Färben von keratinischen Fasern daher dadurch gekennzeichnet, dass es - bezogen auf das Gesamtgewicht des Mittels - einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

Die Farbstoffe der allgemeinen Formel (I) können beispielsweise nach einem Verfahren hergestellt werden, wie es in WO 2002/100369 A2 beschrieben ist.

So kann beispielsweise das Edukt 2-Aminothiazol in konzentrierter Schwefelsäure mit Nitrosylschwefelsäure in das Diazoniumion überführt werden:

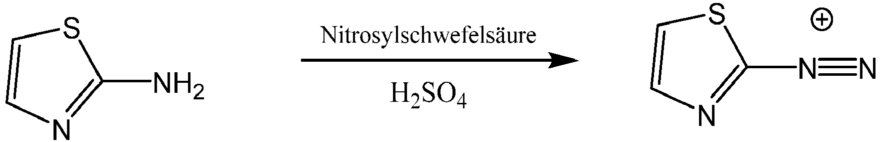

Das reaktive Diazoniumion geht dann mit dimeren Anilin-Derivaten eine doppelte Azokupplungsreaktion ein:

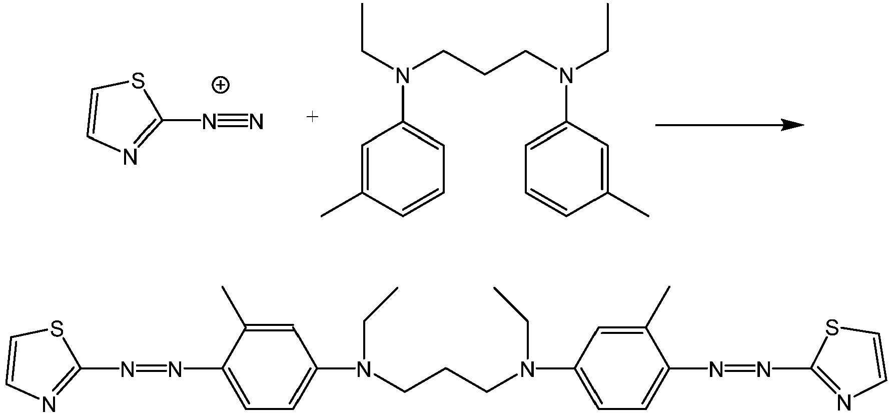

Das in der Azokupplungsreaktion eingesetzte Edukt N,N'Diethyl-N,N'-bis(3-methylphenyl)-1,3-propandiamin und die hierzu analogen Verbindungen können beispielsweise hergestellt werden, wie es in US 4 562 249 beschrieben wurde.

Der in der Azokupplungsreaktion entstehende neutrale dimere Farbstoff kann dann schließlich mit Quaternierungsmitteln doppelt quaterniert werden. Die Quaternierungsreaktion wird bevorzugt in einem polaren aprotischen Lösungsmittel (wie beispielsweise DMSO, DMF etc.) durchgeführt. Als Quaternierungsmittel kommen zum Beispiel Dimethylsulfat, Methylbromid oder p-Toluolsulfonat in Frage.

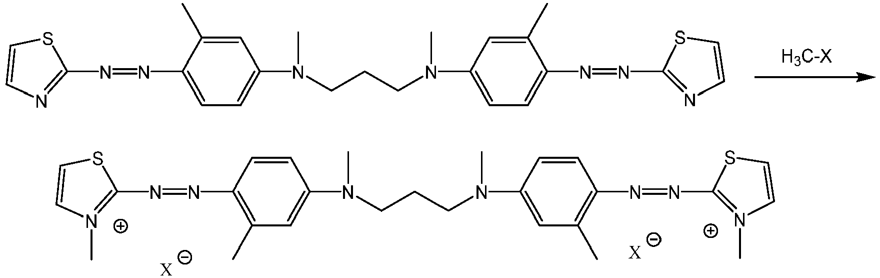

Eine zentrale Aufgabenstellung der vorliegenden Anmeldung lag in der Bereitstellung von Färbemitteln mit direktziehenden Farbstoffen, welche eine hohe Oxidationsstabilität besitzen damit die gleichzeitige starke Aufhellung und Färbung von keratinischen Fasern ermöglichen. Überraschenderweise zeigten die direktziehenden Farbstoffe der Formel (I) eine besonders hohe Stabilität gegenüber einem Gemisch aus Wasserstoffperoxid und Persulfaten. Um die simultane Färbung und Aufhellung zu ermöglichen, enthalten die erfindungsgemäßen Mittel daher in einer weiteren bevorzugten Ausführungsform zusätzlich Wasserstoffperoxid.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 12,5 Gew.-%, besonders bevorzugt 2,5 bis 10 Gew.-% und insbesondere 4,0 bis 9,0 Gew.-% Wasserstoffperoxid, jeweils bezogen auf das Gesamtgewicht des Mittels, enthalten.

In einer weiteren besonders bevorzugten Ausführungsform ist ein erfindungsgemäßes Mittel dadurch gekennzeichnet, dass es - bezogen auf das Gesamtgewicht des Mittels - 0,5 bis 12,5 Gew.-%, bevorzugt 2,5 bis 10 Gew.-% und insbesondere 4,0 bis 9,0 Gew.-% Wasserstoffperoxid enthält.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

In einer weiteren besonders bevorzugten Ausführungsform ist ein erfindungsgemäßes Mittel dadurch gekennzeichnet, dass es mindestens ein Persulfat aus der Gruppe Ammoniumperoxodisulfat, Kaliumperoxodisulfat und Natriumperoxodisulfat enthält.

Bei Ammoniumperoxodisulfat handelt es sich um eine Verbindung der Formel (NH₄)₂S₂O₈. Bei Kaliumperoxodisulfat handelt es sich um eine Verbindung der Formel K₂S₂O₈. Bei Natrjiumperoxodisulfat handelt es sich um eine Verbindung der Formel Na₂S₂O₈.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

In einer weiteren besonders bevorzugten Ausführungsform ist ein erfindungsgemäßes Mittel dadurch gekennzeichnet, dass es - bezogen auf das Gesamtgewicht des Mittels - ein oder mehrere Persulfate aus der Gruppe Ammoniumperoxodisulfat, Kaliumperoxodisulfat und Natriumperoxodisulfat in einer Gesamtmenge von 0,5 bis 20 Gew.-%, vorzugsweise von 1,0 bis 12,5 Gew.-%, weiter bevorzugt von 2,5 bis 10 Gew.-% und besonders bevorzugt von 3,0 bis 6,0 Gew.-% enthält.

Die erfindungsgemäßen direktziehenden Farbstoffe der allgemeinen Formel (I) haben sich gegenüber bestimmten Gemischen aus Ammoniumperoxodisulfat, Kaliumperoxodisulfat und Natriumperoxodisulfat als besonders stabil erwiesen.

In einer weiteren besonders bevorzugten Ausführungsform ist ein erfindungsgemäßes Mittel dadurch gekennzeichnet,, dass es - bezogen auf das Gesamtgewicht des Mittels -
- 0,2 bis 13,5 Gew.-%, bevorzugt 0,5 bis 1,5 Gew.-%, Ammoniumperoxodisulfat
- 0,5 bis 4,5 Gew.-%, bevorzugt 1,5 bis 2,5 Gew.-%, Kaliumperoxodisulfat und
- 0,2 bis 1,8 Gew.-%, bevorzugt 0,4 bis 0,8 Gew.-%, Natriumperoxodisulfat enthält.

Das erfindungsgemäße Farbänderungsmittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

n einer weiteren bevorzugten Ausführungsform enthalten die erfindungsgemäßen Mittel neben der Verbindung der Formel (I) zusätzlich mindestens einen weiteren direktziehenden Farbstoff. Durch die Kombination mit weiteren kationischen direktziehenden Farbstoffen lässt sich das erzielbare Nuancenspektrum erweitern, und die Färbeeigenschaften lassen sich noch weiter verbessern.

In einer weiteren ganz besonders bevorzugten Ausführungsform ist ein erfindungsgemäßes Mittel daher dadurch gekennzeichnet, dass es mindestens eine Si02-Verbindung aus der Gruppe der Kieselsäuren, der Silikate, der Wassergläser und/oder der Alkalimetallmetasilikate enthält.

Zur weiteren Nuancierung kann das erfindungsgemäße Mittel zur Farbveränderung von keratinischen Fasern zusätzlich auch noch mindestens einen weiteren direktziehenden Farbstoff enthalten. Direktziehende Farbstoffe können in anionische, kationische und nichtionische direktziehende Farbstoffe unterteilt werden.

Die direktziehenden Farbstoffe sind vorzugsweise ausgewählt aus den kationischen direktziehenden Farbstoffen, da diese mit den Farbstoffen der allgemeinen Formel (I) gut kompatibel sind.

In einer weiteren besonders bevorzugten Ausführungsform ist ein erfindungsgemäßes Mittel dadurch gekennzeichnet, dass es zusätzlich mindestens einen weiteren kationischen direktziehenden Farbstoff enthält, der von den Farbstoffen der Formel (I) verschieden ist.

Unter kationischen Farbstoffen werden in diesem Zusammenhang Farbstoffe verstanden, die mindestens eine positive Ladung tragen.

Als besonders gut kompatibel haben sich ein oder mehrere Farbstoffe aus der Gruppe Basic Yellow 87, Basic Orange 31, Basic Red 51, Basic Violet 2 und Cationic Blue 347 erwiesen.

Ganz besonders gut kompatibel sind die Farbstoffe der Formel (I) mit den kationischen Azofarbstoffen Basic Orange 31 und Basic Red 51. Durch Kombination eines Farbstoffes der Formel (I) mit Basic Orange 31 und/oder Basice Red 51 können - außer rein gelben Nuancen - Nuancen im gesamten Farbspektrum erzeugt werden.

In einer weiteren besonders bevorzugten Ausführungsform ist ein erfindungsgemäßes Mittel dadurch gekennzeichnet, dass es zusätzlich Basic Orange 31 und/oder Basic Red 51 enthält.

Das erfindungsgemäße Mittel kann aber auch zusätzlich mindestens einen nichtionischen direktziehenden Farbstoff enthalten. Dieser kann ausgewählt sein aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethyl-amino-3-nitrobenzoesäure und 2-Chlor-6-ethylamino-4-nitrophenol.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind. Die erfindungsgemäßen Mittel können weiterhin auch zusammen mit Oxidationsfärbemitteln eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die zusätzlichen direktziehenden Farbstoffe, Entwicklerkomponenten und Kupplerkomponenten werden bevorzugt jeweils in einem Anteil von 0,0001 bis 5,0 Gew.-%, vorzugsweise 0,001 bis 3,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Die Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Die Mittel des ersten Erfindungsgegenstands können in Verfahren zum Färben und auch in Verfahren zum gleichzeitigen Blondieren bzw. Aufhellen und Färben menschlicher Haare genutzt werden.

Die erfindungsgemäßen Mittel können als Einkomponentenmittel oder als Mehrkomponentenmittel wie Zweikomponenten Mittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Unter dem erfindungsgemäßen Mittel zur Farbveränderung von keratinischen Fasern wird immer das anwendungsbereite Mittel verstanden.

Wird das erfindungsgemäße Mittel dem Anwender in Form eines Einkomponenten-Mittels zur Verfügung gestellt, dann muss das anwendungsbereite Mittel nicht erst hergestellt werden, sondern kann direkt aus dem Behältnis, in dem es konfektioniert wurde, entnommen und auf die keratinischen Fasern appliziert werden.

Bei Blondiermitteln handelt es sich jedoch üblicherweise um Zweikomponentenprodukte, bei welchen eine oxidationsmittelhaltige Komponente (A1) kurz vor der Anwendung mit einem (Alkalisierungs-)Mittel (A2) vermischt und diese anwendungsbereite Mischung auf die Haare aufgetragen wird.

In diesem Fall handelt es sich bei dem erfindungsgemäßen Mittel um das anwendungsbereite Mittel, welches kurz vor der Anwendung durch Vermischen von (A1) und (A2) hergestellt wurde.

Hierbei können die direktziehenden Farbstoffe der allgemeinen Formel (I) in der Komponente (A1) (d.h. zusammen mit dem Oxidationsmittel) oder aber in der Komponente (A2) (zusammen mit dem Alkalisierungsmittel) konfektioniert werden.

Es ist ebenfalls möglich und erfindungsgemäß, wenn das anwendungsbereite Mittel kurz vor der Anwendung auf den menschlichen Haaren durch Vermischen von 3 Komponenten hergestellt wird, wobei
- die Komponente (A1) mindestens einen direktziehenden Farbstoff der allgemeinen Formel (I) und mindestens ein Alkalisierungsmittel
- die Komponente (A2) mindestens ein erstes Oxidationsmittel (z.B. Wasserstoffperoxid) und
- die Komponente (A3) mindestens ein zweites Oxidationsmittel (z.B. ein oder mehrere Poroxodisulfatsalze) enthält.

Während der Einwirkzeit der Mittel auf der Faser kann es vorteilhaft sein, den Aufhellvorgang oder Mattiervorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die behandelte Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Bezüglich weiterer bevorzugter Ausführungsformen der erfindungsgemäßen Verwendung und Verfahren gilt mutatis mutandis das zu den erfindungsgemäßen Mitteln Gesagte.

### Beispiele

### Direktzieher 1: 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)

Der Farbstoff DZ 1 wurde nach einem Verfahren synthetisiert, wie es in den Dokumenten WO 2002/100369 A2 und US 4 562 249 beschrieben ist.

### DZ 1 (erfindungsgemäß)

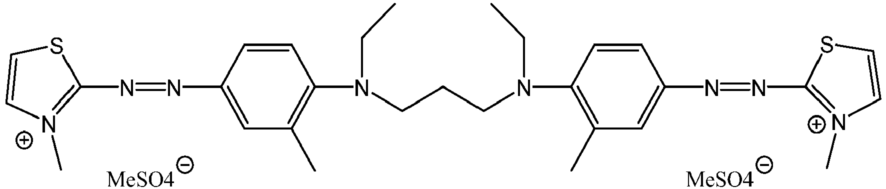

### Direkzieher 2: 3-Methyl-2-(2-{4-[methyl({3-[methyl({4-[2-(3-methyl-1,3-thiazol-3-ium-2-yl)diazen-1-yl]phenyl})amino]propyl})aminolphenyl}diazen-1-yl)-1,3-thiazol-3-ium, Di(methylsulfat)

### (DZ 1, erfindungsgemäß)

Der Farbstoff DZ 2 wurde nach einem Verfahren synthetisiert, wie es in den Dokumenten WO 2002/100369 A2 und US 3291788 beschrieben ist.

Als Edukte wurden 2-Aminothiazol und N,N'-Dimethyl-N,N'-diphenyl-propan-1,3-diamin eingesetzt (Azokupplungsreaktion in wässriger, schwefelsauerer Lösung mit Nitrosylschwefelsäure). Der in dieser Azokupplungsreaktion entstehende neutrale Farbstoff wurde im Anschluss daran quaterniert (beispielsweise mit dem Quaternierungsmittel Dimethylsulfat in einem polaren, aprotischen Lösungsmittel wie Dimethylformamid oder Dimethylsulfoxid).

### DZ 2 (Vergleich)

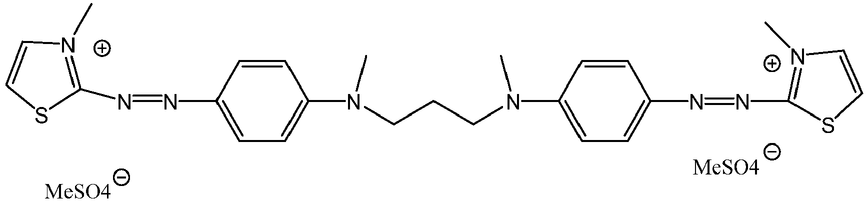

### Direktzieher 3: 5-[2-(4-{[3-({4-[2-(1,4-Dimethyl-1H-1,2,4-triazol-4-ium-5-yl)diazen-1-yl]phenyl}(methyl)amino)propyl](methyl)amino}phenyl)diazen-1-yl]-1,4-dimethyl-1H-1 ,2,4-triazol-4-ium Di(methylsulfat)

Der Farbstoff DZ 3 wurde nach einem Verfahren synthetisiert, wie es in dem Dokument WO 2002/100369 A2 beschrieben ist.

### DZ 3 (Vergleich)

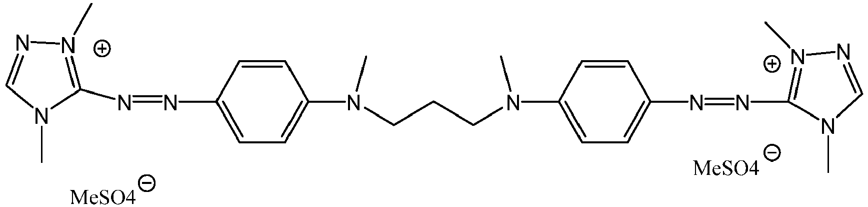

### Färbebeispiele

### Formulierungen

Es wurden die folgenden Färbecremes hergestellt (alle Angaben in Gew.-%, Aktivsubstanz)

| **Farbcreme** | **E** | **V1** | **V2** |
|---|---|---|---|
| Cetearylalkohol | 3,5 | 3,5 | 3,5 |
| C12-C18 Fettalkohole | 0,9 | 0,9 | 0,9 |
| Ceteareth-20 | 0,1 | 0,1 | 0,1 |
| Quaternium-87 | 1,0 | 1,0 | 1,0 |
| Natrium Laurethsulfat | 1,9 | 1,9 | 1,9 |
| Ammoniumsulfat | 0,2 | 0,2 | 0,23 |
| Etidronsäure | 0,1 | 0,1 | 0,1 |
| Ammoniumhydroxid | 0,1 | 0,1 | 0,1 |
| Natriumsilikat | 0,2 | 0,2 | 0,2 |
| Ammoniak (25 %ige wässrige Lösung) | 2,5 | 2,5 | 2,5 |
| DZ1 (erfindungsgemäß) | 0,7 | -- | --- |
| DZ 2 (Vergleich) | --- | 0,7 | --- |
| DZ 3 (Vergleich) | --- | --- | 0,7 |
| Wasser | ad 100 | ad 100 | ad 100 |

Die direktziehenden Farbstoffe DZ1, DZ2 und DZ3 wurden jeweils in wenig Wasser vorgelöst und mit den restlichen Formulierungsbestandteilen vermischt.

Es wurde die folgende Entwickleremulsion hergestellt (Gesamtmenge 36 g)

| **Entwickleremulsion** | |
|---|---|
| Natriumhydroxid | 0,2 |
| Etidronsäure | 0,5 |
| Natriumlaurethsulfat | 0,7 |
| Acrylat Compolymer | 4,3 |
| Wasserstoffperoxid (50 %ige Lösung) | 8,0 |
| Wasser | 22,0 g |

Es wurde das folgende Blondierpulver hergestellt (Gesamtmenge 13,6 g)

| **Entwickleremulsion** | |
|---|---|
| Ammoniumpersulfat | 1,8 |
| Kalumpersulfat | 5,8 |
| Natriumpersulfat | 2,1 |
| Natriumsilikat | 3,8 |
| Dinatrium EDTA | 0,1 |

Jede der Farbcremes E, V1 und V2 wurde im Gewichtsverhältnis 1:1:1 mit der Entwickleremulsion und mit dem Blondierpulver vermischt.

### Ausfärbungen

Jeweils 1,8 g der Färbecreme wurden auf eine ca. 6 cm lange Strähne Menschenhaares (Kerling Euronaturhaar,80 % ergraut) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Nach dem Trocknen wurden die Färbung und die Farbintensität der Strähnen visuell unter der Tageslichtlampe beurteilt.

| | Formulierung | pH-Wert | Farbnuance | Farbintensität |
|---|---|---|---|---|
| E1 | DZ1 (erfindungsgemäß) | 9,5 | tiefviolett | ++++ |
| V1 | DZ 2 (Vergleich) | 9,5 | blass violett | + |
| V2 | DZ 3 (Vergleich) | 9,5 | blass rosa | ++ |

| | | | | |
|---|---|---|---|---|
| Farbintensität: + = schlecht +++ = mittel +++++ = sehr gut | | | | |

## Patentansprüche

1. Mittel zur Farbveränderung von keratinischen Fasern, insbesondere menschlichen Haaren, enthaltend in einem kosmetischen Träger
(a) mindestens einen direktziehenden Farbstoff der Formel (I), 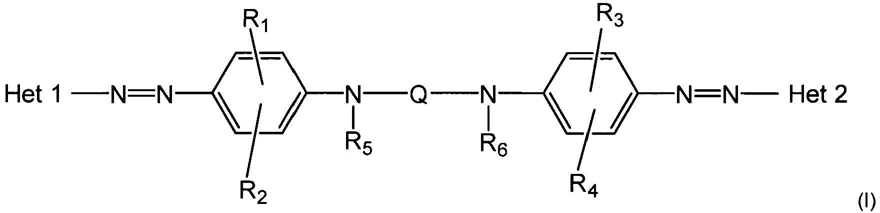 wobei
Het1, Het2 unabhängig voneinander für eine der Strukturen (II), (III), (IV), (V), (VI) oder (VII) stehen, 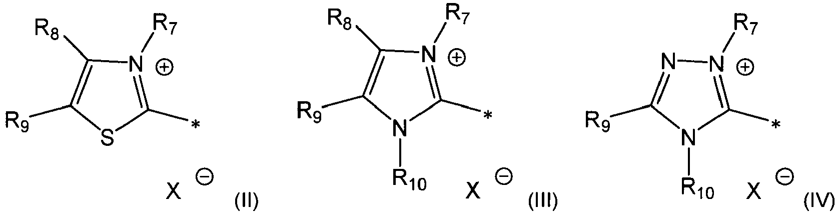 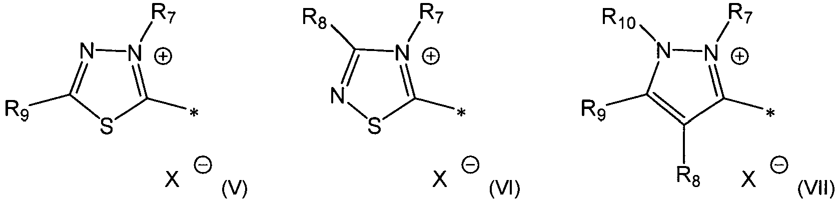
R1, R3 unabhängig voneinander für eine C₁-C₆-Alkylgruppe oder für eine C₁-C₆-Alkoxygruppe stehen,
R2, R4 beide für ein Wasserstoffatom, stehen,
R5, R6 unabhängig voneinander für ein Wasserstoffatom, eine C₁-C₆-Alkylgruppe oder für eine C₂-C₆-Alkenylgruppe stehen,
Q für eine Gruppierung der Formel (VIII) steht,
*-(CH₂)n-* VIII
n für eine ganze Zahl von 3 bis 6 steht,
R7, R10 jeweils unabhängig voneinander für eine C₁-C₆-Alkylgruppe oder für eine C₂-C₆-Alkenylgruppe stehen,
R8, R9 jeweils unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen,
X- für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat, steht.

2. Mittel nach Anspruch 1, **dadurch gekennzeichnet, dass** es (a) mindestens einen direktziehenden Farbstoff der allgemeinen Formel (I) enthält, in der
Het1, Het2 unabhängig voneinander jeweils für eine der Strukturen (II), (III) oder (IV) stehen

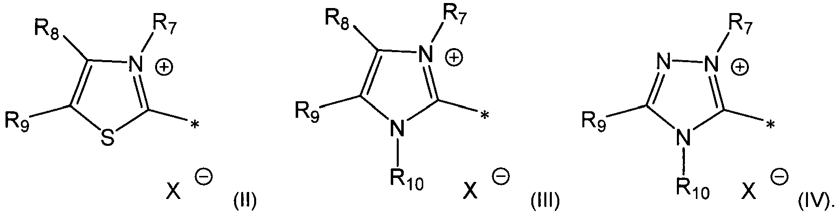

3. Mittel nach einem der Ansprüche 1 bis 2, **dadurch gekennzeichnet, dass** es (a) mindestens einen direktziehenden Farbstoff der allgemeinen Formel (I) enthält, in der
R5, R6 unabhängig voneinander für Wasserstoff oder für eine C₁-C₆-Alkylgruppe stehen.

4. Mittel nach einem der Ansprüche 1 bis 4 3, **dadurch gekennzeichnet, dass** es (a) mindestens einen direktziehenden Farbstoff der allgemeinen Formel (I) enthält, in der
R7, R10 jeweils unabhängig voneinander für eine Methylgruppe oder eine Ethylgruppe stehen,
R8, R9 für ein Wasserstoffatom stehen.
*-(CH₂)n- *

5. Mittel nach einem der Ansprüche 1 bis 4, **dadurch gekennzeichnet, dass** es (a) mindestens einen direktziehenden Farbstoff der allgemeinen Formel (I) enthält, der ausgewählt ist aus
- Salzen des 3-Methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-iums
- Salzen des 3-Methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-iums
- Salzen des 3-Methyl-2-[2-(3-methyl-4-{[4-({2-methyl-4-[2-(3-methyl-1,3-thiazol-3-ium-2-yl)diazen-1-yl]phenyl}amino)butyl]amino}phenyl)diazen-1-yl]-1,3-thiazol-3-iums
- Salzen des 3-Methyl-2-[2-(2-methyl-4-{[4-({3-methyl-4-[2-(3-methyl-1,3-thiazol-3-ium-2-yl)diazen-1-yl]phenyl}amino)butyl]amino}phenyl)diazen-1-yl]-1,3-thiazol-3-iums
- Salzen des 3-Methyl-2-[2-(3-methyl-4-{[5-({2-methyl-4-[2-(3-methyl-1,3-thiazol-3-ium-2-yl)diazen-1-yl]phenyl}amino)pentyl]amino}phenyl)diazen-1-yl]-1,3-thiazol-3-iums
- Salzen des 3-Methyl-2-[2-(2-methyl-4-{[5-({3-methyl-4-[2-(3-methyl-1,3-thiazol-3-ium-2-yl)diazen-1-yl]phenyl}amino)pentyl]amino}phenyl)diazen-1-yl]-1,3-thiazol-3-iums
- Salzen des 3-Methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-iums
- Salzen des 3-Methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-iums
- Salzen des 3-Methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-iums
- Salzen des 3-Methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-iums
- Salzen des 3-Methyl-2-(2-{3-methyl-4-[methyl({4-[methyl({2-methyl-4-[2-(3-methyl-1,3-thiazol-3-ium-2-yl)diazen-1-yl]phenyl})amino]butyl})amino]phenyl}diazen-1-yl)-1,3-thiazol-3-iums
- Salzen des 3-Methyl-2-(2-{2-methyl-4-[methyl({4-[methyl({3-methyl-4-[2-(3-methyl-1,3-thiazol-3-ium-2-yl)diazen-1-yl]phenyl})amino]butyl})amino]phenyl}diazen-1-yl)-1,3-thiazol-3-iums
- Salzen des 3-Methyl-2-(2-{3-methyl-4-[methyl({5-[methyl({2-methyl-4-[2-(3-methyl-1,3-thiazol-3-ium-2-yl)diazen-1-yl]phenyl})amino]pentyl})amino]phenyl}diazen-1-yl)-1,3-thiazol-3-iums
- Salzen des 3-Methyl-2-(2-{2-methyl-4-[methyl({5-[methyl({3-methyl-4-[2-(3-methyl-1,3-thiazol-3-ium-2-yl)diazen-1-yl]phenyl})amino]pentyl})amino]phenyl}diazen-1-yl)-1,3-thiazol-3-iums
- Salzen des 2-[2-(3-Methoxy-4-{[3-({2-methoxy-4-[2-(3-methyl-1,3-thiazol-3-ium-2-yl)diazen-1-yl]phenyl}amino)propyl]amino}phenyl)diazen-1-yl]-3-methyl-1,3-thiazol-3-iums
- Salzen des 2-[2-(2-Methoxy-4-{[3-({3-methoxy-4-[2-(3-methyl-1,3-thiazol-3-ium-2-yl)diazen-1-yl]phenyl}amino)propyl]amino}phenyl)diazen-1-yl]-3-methyl-1,3-thiazol-3-iums
- Salzen des 2-[2-(3-Methoxy-4-{[4-({2-methoxy-4-[2-(3-methyl-1,3-thiazol-3-ium-2-yl)diazen-1-yl]phenyl}amino)butyl]amino}phenyl)diazen-1-yl]-3-methyl-1,3-thiazol-3-iums
- Salzen des 2-[2-(2-Methoxy-4-{[4-({3-methoxy-4-[2-(3-methyl-1,3-thiazol-3-ium-2-yl)diazen-1-yl]phenyl}amino)butyl]amino}phenyl)diazen-1-yl]-3-methyl-1,3-thiazol-3-iums
- Salzen des 2-[2-(3-Methoxy-4-{[5-({2-methoxy-4-[2-(3-methyl-1,3-thiazol-3-ium-2-yl)diazen-1-yl]phenyl}amino)pentyl]amino}phenyl)diazen-1-yl]-3-methyl-1,3-thiazol-3-iums
- Salzen des 2-[2-(2-Methoxy-4-{[5-({3-methoxy-4-[2-(3-methyl-1,3-thiazol-3-ium-2-yl)diazen-1-yl]phenyl}amino)pentyl]amino}phenyl)diazen-1-yl]-3-methyl-1,3-thiazol-3-iums
- Salzen des 2-[2-(3-Methoxy-4-{[3-({2-methoxy-4-[2-(3-methyl-1,3-thiazol-3-ium-2-yl)diazen-1-yl]phenyl}(methyl)amino)propyl](methyl)amino}phenyl)diazen-1-yl]-3-methyl-1,3-thiazol-3-iums
- Salzen des 2-[2-(2-Methoxy-4-{[3-({3-methoxy-4-[2-(3-methyl-1,3-thiazol-3-ium-2-yl)diazen-1-yl]phenyl}(methyl)amino)propyl](methyl)amino}phenyl)diazen-1-yl]-3-methyl-1,3-thiazol-3-iums
- Salzen des 2-[2-(3-Methoxy-4-{[4-({2-methoxy-4-[2-(3-methyl-1,3-thiazol-3-ium-2-yl)diazen-1-yl]phenyl}(methyl)amino)butyl](methyl)amino}phenyl)diazen-1-yl]-3-methyl-1,3-thiazol-3-iums
- Salzen des 2-[2-(2-Methoxy-4-{[4-({3-methoxy-4-[2-(3-methyl-1,3-thiazol-3-ium-2-yl)diazen-1-yl]phenyl}(methyl)amino)butyl](methyl)amino}phenyl)diazen-1-yl]-3-methyl-1,3-thiazol-3-iums
- Salzen des 2-[2-(3-Methoxy-4-{[5-({2-methoxy-4-[2-(3-methyl-1,3-thiazol-3-ium-2-yl)diazen-1-yl]phenyl}(methyl)amino)pentyl](methyl)amino}phenyl)diazen-1-yl]-3-methyl-1,3-thiazol-3-iums
- Salzen des 2-[2-(2-Methoxy-4-{[5-({3-methoxy-4-[2-(3-methyl-1,3-thiazol-3-ium-2-yl)diazen-1-yl]phenyl}(methyl)amino)pentyl](methyl)amino}phenyl)diazen-1-yl]-3-methyl-1,3-thiazol-3-iums
- Salzen des 5-[2-(4-{[3-({4-[2-(1,4-Dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-y]-2-methylphenyllamino)butyl]aminol-3-methylphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-3-methylphenyl}amino)butyl]amino}-2-methylphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-2-methylphenyl}amino)pentyl]amino}-3-methylphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-3-methylphenyl)amino)pentyl]amino)-2-methylphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-2-methylphenyl}(methyl)amino)butyl](methyl)amino}-3-methylphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-3-methylphenyl}(methyl)amino)butyl](methyl)amino}-2-methylphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-2-methylphenyl}(methyl)amino)pentyl](methyl)amino}-3-methylphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-3-methylphenyl}(methyl)amino)pentyl](methyl)amino}-2-methylphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-2-methoxyphenyl}amino)propyl]amino}-3-methoxyphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-3-methoxyphenyl}amino)propyl]amino}-2-methoxyphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-2-methoxyphenyl}amino)butyl]amino}-3-methoxyphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-3-methoxyphenyl}amino)butyl]amino}-2-methoxyphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-2-methoxyphenyl}amino)pentyl]amino}-3-methoxyphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-3-methoxyphenyl}amino)pentyl]amino}-2-methoxyphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-2-methoxyphenyl}(methyl)amino)propyl](methyl)amino}-3-methoxyphenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]-3-methoxyphenyl}(methyl)amino)propyl](methyl)amino}-2-methoxyphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-2-methoxyphenyl}(methyl)amino)butyl](methyl)amino}-3-methoxyphenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]-3-methoxyphenyl}(methyl)amino)butyl](methyl)amino}-2-methoxyphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-2-methoxyphenyl}(methyl)amino)pentyl](methyl)amino}-3-methoxyphenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]-3-methoxyphenyl}(methyl)amino)pentyl](methyl)amino}-2-methoxyphenyl)diazen-1-yl]-1,4-dimethyl-1H-1,2,4-triazol-4-iums

6. Mittel nach einem der Ansprüche 1 bis 5, **dadurch gekennzeichnet, dass** es - bezogen auf das Gesamtgewicht des Mittels - einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

7. Mittel nach einem der Ansprüche 1 bis 6, **dadurch gekennzeichnet, dass** es - bezogen auf das Gesamtgewicht des Mittels - 0,5 bis 12,5 Gew.-%, bevorzugt 2,5 bis 10 Gew.-% und insbesondere 4,0 bis 9,0 Gew.-% Wasserstoffperoxid enthält.

8. Mittel nach einem der Ansprüche 1 bis 7, **dadurch gekennzeichnet, dass** es mindestens ein Persulfat aus der Gruppe Ammoniumperoxodisulfat, Kaliumperoxodisulfat und Natriumperoxodisulfat enthält.

9. Mittel nach einem der Ansprüche 1 bis 8, **dadurch gekennzeichnet, dass** es - bezogen auf das Gesamtgewicht des Mittels - ein oder mehrere Persulfate aus der Gruppe Ammoniumperoxodisulfat, Kaliumperoxodisulfat und Natriumperoxodisulfat in einer Gesamtmenge von 0,5 bis 20 Gew.-%, vorzugsweise von 1,0 bis 12,5 Gew.-%, weiter bevorzugt von 2,5 bis 10 Gew.-% und besonders bevorzugt von 3,0 bis 6,0 Gew.-% enthält.

10. Mittel nach einem der Ansprüche 1 bis 9, dadurch gekenneichnet, dass es - bezogen auf das Gesamtgewicht des Mittels -
- 0,2 bis 13,5 Gew.-%, bevorzugt 0,5 bis 1,5 Gew.-%, Ammoniumperoxodisulfat
- 0,5 bis 4,5 Gew.-%, bevorzugt 1,5 bis 2,5 Gew.-%, Kaliumperoxodisulfat und
- 0,2 bis 1,8 Gew.-%, bevorzugt 0,4 bis 0,8 Gew.-%, Natriumperoxodisulfat enthält.

11. Mittel nach einem der Ansprüche 1 bis 10, **dadurch gekennzeichnet, dass** es mindestens eine SiO₂-Verbindung aus der Gruppe der Kieselsäuren, der Silikate, der Wassergläser und/oder der Alkalimetallmetasilikate enthält.

12. Mittel nach einem der Ansprüche 1 bis 11, **dadurch gekennzeichnet, dass** es zusätzlich mindestens einen weiteren kationischen direktziehenden Farbstoff enthält, der von den Farbstoffen der Formel (I) verschieden ist.

13. Mittel nach einem der Ansprüche 1 bis 12, **dadurch gekennzeichnet, dass** es zusätzlich Basic Orange 31 und/oder Basic Red 51 enthält.

## Claims

1. An agent for changing the color of keratin fibers, in particular human hair, said agent containing, in a cosmetic carrier,
(a) at least one substantive dye of formula (I), 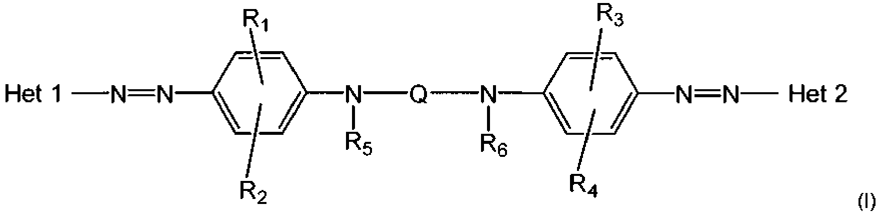 where Het1 and Het2 represent, independently of one another, one of structures (II), (III), (IV), (V), (VI) or (VII), 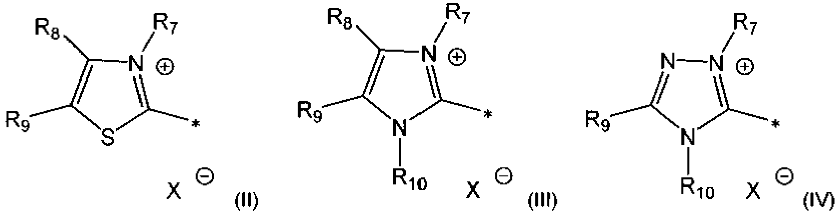 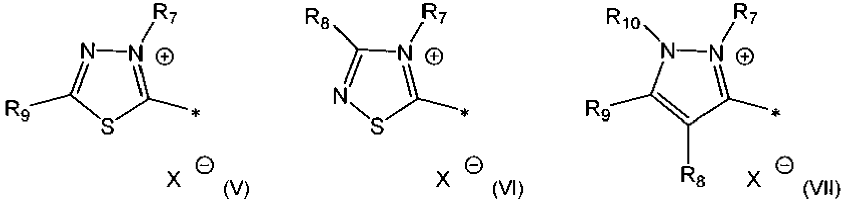
R1 and R3 represent, independently of one another, a C₁-C₆ alkyl group or a C₁-C₆ alkoxyl group,
R2 and R4 both represent a hydrogen atom,
R5 and R6 represent, independently of one another, a hydrogen atom, a C₁-C₆ alkyl group or a C₂-C₆ alkenyl group,
Q represents a grouping of formula (VIII), *-(CH₂)n-* (VIII)
n represents an integer from 3 to 6,
R7 and R10 each represent, independently of one another, a C₁-C₆ alkyl group or a C₂-C₆ alkenyl group,
R8 and R9 each represent, independently of one another, a hydrogen atom, a C₁-C₆ alkyl group, a halogen atom from the group chlorine, bromine, fluorine or iodine, a C₁-C₆ alkoxyl group, or a nitro group,
X- represents a physiologically acceptable anion, preferably from the group chloride, bromide, iodide, methyl sulfate, methyl sulfonate, p-toluene sulfonate, acetate, hydrogen sulfate, ½ sulfate or ½ tetrachlorozincate.

2. The agent according to claim 1, **characterized in that** said agent contains (a) at least one substantive dye of general formula (I), in which
Het1 and Het2 represent, independently of one another, one of structures (II), (III) or

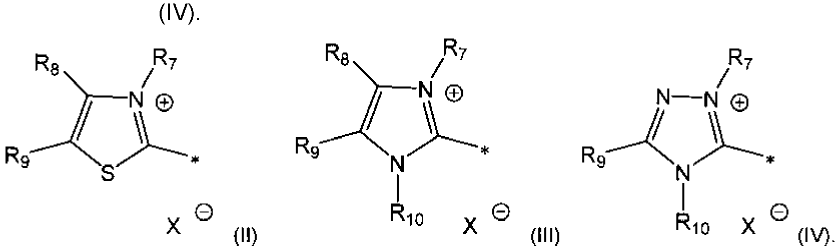

3. The agent according to one of claims 1 or 2, **characterized in that** said agent contains (a) at least one substantive dye of general formula (I), in which
R5 and R6 represent, independently of one another, a hydrogen atom or a C₁-C₆ alkyl group.

4. The agent according to one of claims 1 to 3, **characterized in that** said agent contains (a) at least one substantive dye of general formula (I), in which
R7 and R10 represent, independently of one another, a methyl group or an ethyl group, and
R8 and R9 represent a hydrogen atom.

5. The agent according to one of claims 1 to 4, **characterized in that** said agent contains (a) at least one substantive dye of general formula (I), which dye is selected from
- salts of 3-methyl-2-[2-(3-methyl-4-{[3-({2-methyl-4-[2-(3-methyl-1,3-thiazol-3-ium-2-yl)diazen-1-yl]phenyl}amino)propyl]amino}phenyl)diazen-1-yl]-1,3-thiazol-3-ium
- salts of 3-methyl-2-[2-(2-methyl-4-{[3-({3-methyl-4-[2-(3-methyl-1,3-thiazol-3-ium-2-yl)diazen-1-yl]phenyl}amino)propyl]amino}phenyl)diazen-1-yl]-1,3-thiazol-3-ium
- salts of 3-methyl-2-[2-(3-methyl-4-{[4-({2-methyl-4-[2-(3-methyl-1,3-thiazol-3-ium-2-yl)diazen-1-yl]phenyl}amino)butyl]amino}phenyl)diazen-1-yl]-1,3-thiazol-3-ium
- salts of 3-methyl-2-[2-(2-methyl-4-{[4-({3-methyl-4-[2-(3-methyl-1,3-thiazol-3-ium-2-yl)diazen-1-yl]phenyl}amino)butyl]amino}phenyl)diazen-1-yl]-1,3-thiazol-3-ium
- salts of 3-methyl-2-[2-(3-methyl-4-{[5-({2-methyl-4-[2-(3-methyl-1,3-thiazol-3-ium-2-yl)diazen-1-yl]phenyl}amino)pentyl]amino}phenyl)diazen-1-yl]-1,3-thiazol-3-ium
- salts of 3-methyl-2-[2-(2-methyl-4-{[5-({3-methyl-4-[2-(3-methyl-1,3-thiazol-3-ium-2-yl)diazen-1-yl]phenyl}amino)pentyl]amino}phenyl)diazen-1-yl]-1,3-thiazol-3-ium
- salts of 3-methyl-2-(2-{3-methyl-4-[methyl({3-[methyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium
- salts of 3-methyl-2-(2-{3-methyl-4-[ethyl({3-[ethyl({2-methyl-4-[2-(3-methyl-1,3-thiazol-3-ium-2-yl)diazen-1-yl]phenyl})amino]propyl})amino]phenyl}diazen-1-yl)-1,3-thiazol-3-ium
- salts of 3-methyl-2-(2-{2-methyl-4-[methyl({3-[methyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium
- salts of 3-methyl-2-(2-{2-methyl-4-[ethyl({3-[ethyl({3-methyl-4-[2-(3-methyl-1,3-thiazol-3-ium-2-yl)diazen-1-yl]phenyl})amino]propyl})amino]phenyl}diazen-1-yl)-1,3-thiazol-3-ium
- salts of 3-methyl-2-(2-{3-methyl-4-[methyl({4-[methyl({2-methyl-4-[2-(3-methyl-1,3-thiazol-3-ium-2-yl)diazen-1-yl]phenyl})amino]butyl})amino]phenyl}diazen-1-yl)-1,3-thiazol-3-ium
- salts of 3-methyl-2-(2-{2-methyl-4-[methyl({4-[methyl({3-methyl-4-[2-(3-methyl-1,3-thiazol-3-ium-2-yl)diazen-1-yl]phenyl})amino]butyl})amino]phenyl}diazen-1-yl)-1,3-thiazol-3-ium
- salts of 3-methyl-2-(2-{3-methyl-4-[methyl({5-[methyl({2-methyl-4-[2-(3-methyl-1,3-thiazol-3-ium-2-yl)diazen-1-yl]phenyl})amino]pentyl})amino]phenyl}diazen-1-yl)-1,3-thiazol-3-ium
- salts of 3-methyl-2-(2-{2-methyl-4-[methyl({5-[methyl({3-methyl-4-[2-(3-methyl-1,3-thiazol-3-ium-2-yl)diazen-1-yl]phenyl})amino]pentyl})amino]phenyl}diazen-1-yl)-1,3-thiazol-3-ium
- salts of 2-[2-(3-methoxy-4-{[3-({2-methoxy-4-[2-(3-methyl-1,3-thiazol-3-ium-2-yl)diazen-1-yl]phenyl}amino)propyl]amino}phenyl)diazen-1-yl]-3-methyl-1,3-thiazol-3-ium
- salts of 2-[2-(2-methoxy-4-{[3-({3-methoxy-4-[2-(3-methyl-1,3-thiazol-3-ium-2-yl)diazen-1-yl]phenyl}amino)propyl]amino}phenyl)diazen-1-yl]-3-methyl-1,3-thiazol-3-ium
- salts of 2-[2-(3-methoxy-4-{[4-({2-methoxy-4-[2-(3-methyl-1,3-thiazol-3-ium-2-yl)diazen-1-yl]phenyl}amino)butyl]amino}phenyl)diazen-1-yl]-3-methyl-1,3-thiazol-3-ium
- salts of 2-[2-(2-methoxy-4-{[4-({3-methoxy-4-[2-(3-methyl-1,3-thiazol-3-ium-2-yl)diazen-1-yl]phenyl}amino)butyl]amino}phenyl)diazen-1-yl]-3-methyl-1,3-thiazol-3-ium
- salts of 2-[2-(3-methoxy-4-{[5-({2-methoxy-4-[2-(3-methyl-1,3-thiazol-3-ium-2-yl)diazen-1-yl]phenyl}amino)pentyl]amino}phenyl)diazen-1-yl]-3-methyl-1,3-thiazol-3-ium
- salts of 2-[2-(2-methoxy-4-{[5-({3-methoxy-4-[2-(3-methyl-1,3-thiazol-3-ium-2-yl)diazen-1-yl]phenyl}amino)pentyl]amino}phenyl)diazen-1-yl]-3-methyl-1,3-thiazol-3-ium
- salts of 2-[2-(3-methoxy-4-{[3-({2-methoxy-4-[2-(3-methyl-1,3-thiazol-3-ium-2-yl)diazen-1-yl]phenyl}(methyl)amino)propyl](methyl)amino}phenyl)diazen-1-yl]-3-methyl-1,3-thiazol-3-ium
- salts of 2-[2-(2-methoxy-4-{[3-({3-methoxy-4-[2-(3-methyl-1,3-thiazol-3-ium-2-yl)diazen-1-yl]phenyl}(methyl)amino)propyl](methyl)amino}phenyl)diazen-1-yl]-3-methyl-1,3-thiazol-3-ium
- salts of 2-[2-(3-methoxy-4-{[4-({2-methoxy-4-[2-(3-methyl-1,3-thiazol-3-ium-2-yl)diazen-1-yl]phenyl}(methyl)amino)butyl](methyl)amino}phenyl)diazen-1-yl]-3-methyl-1,3-thiazol-3-ium
- salts of 2-[2-(2-methoxy-4-{[4-({3-methoxy-4-[2-(3-methyl-1,3-thiazol-3-ium-2-yl)diazen-1-yl]phenyl}(methyl)amino)butyl](methyl)amino}phenyl)diazen-1-yl]-3-methyl-1,3-thiazol-3-ium
- salts of 2-[2-(3-methoxy-4-{[5-({2-methoxy-4-[2-(3-methyl-1,3-thiazol-3-ium-2-yl)diazen-1-yl]phenyl}(methyl)amino)pentyl](methyl)amino}phenyl)diazen-1-yl]-3-methyl-1,3-thiazol-3-ium
- salts of 2-[2-(2-methoxy-4-{[5-({3-methoxy-4-[2-(3-methyl-1,3-thiazol-3-ium-2-yl)diazen-1-yl]phenyl}(methyl)amino)pentyl](methyl)amino}phenyl)diazen-1-yl]-3-methyl-1,3-thiazol-3-ium
- salts of 5-[2-(4-{[3-({4-[2-(1,4-dimethyl-1H-1,2,4-triazol-4-ium-5-yl)diazen-1-yl]-2-methylphenyl}amino)propyl]amino}-3-methylphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-3-methylphenyl}amino)propyl]amino}-2-methylphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-2-methylphenyl}amino)butyl]amino}-3-methylphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-3-methylphenyl}amino)butyl]amino}-2-methylphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-2-methylphenyl}amino)pentyl]amino}-3-methylphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-3-methylphenyl}amino)pentyl]amino}-2-methylphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-2-methylphenyl}(methyl)amino)propyl](methyl)amino}-3-methylphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-3-methylphenyl}(methyl)amino)propyl](methyl)amino}-2-methylphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-2-methylphenyl}(methyl)amino)butyl](methyl)amino}-3-methylphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-3-methylphenyl}(methyl)amino)butyl](methyl)amino}-2-methylphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-2-methylphenyl}(methyl)amino)pentyl](methyl)amino}-3-methylphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-3-methylphenyl}(methyl)amino)pentyl](methyl)amino}-2-methylphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-2-methoxyphenyl}amino)propyl]amino}-3-methoxyphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-3-methoxyphenyl}amino)propyl]amino}-2-methoxyphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-2-methoxyphenyl}amino)butyl]amino}-3-methoxyphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-3-methoxyphenyl}amino)butyl]amino}-2-methoxyphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-2-methoxyphenyl}amino)pentyl]amino}-3-methoxyphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-3-methoxyphenyl}amino)pentyl]amino}-2-methoxyphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-2-methoxyphenyl}(methyl)amino)propyl](methyl)amino}-3-methoxyphenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]-3-methoxyphenyl}(methyl)amino)propyl](methyl)amino}-2-methoxyphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-2-methoxyphenyl}(methyl)amino)butyl](methyl)amino}-3-methoxyphenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]-3-methoxyphenyl}(methyl)amino)butyl](methyl)amino}-2-methoxyphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-2-methoxyphenyl}(methyl)amino)pentyl](methyl)amino}-3-methoxyphenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]-3-methoxyphenyl}(methyl)amino)pentyl](methyl)amino}-2-methoxyphenyl)diazen-1-yl]-1,4-dimethyl-1H-1,2,4-triazol-4-ium

6. The agent according to one of claims 1 to 5, **characterized in that** said agent contains, based on the total weight of the agent, one or more substantive dyes (a) of formula (I) in a total amount of from 0.01 to 4.5 wt.%, preferably from 0.05 to 2.8 wt.%, more preferably from 0.1 to 2.2 wt.% and particularly preferably from 0.2 to 1.2 wt.%.

7. The agent according to one of claims 1 to 6, **characterized in that** said agent contains, based on the total weight of the agent, 0.5 to 12.5 wt.%, preferably 2.5 to 10 wt.% and in particular 4.0 to 9.0 wt.% hydrogen peroxide.

8. The agent according to one of claims 1 to 7, **characterized in that** said agent contains at least one persulfate from the group ammonium peroxodisulfate, potassium peroxodisulfate and sodium peroxodisulfate.

9. The agent according to one of claims 1 to 8, **characterized in that** said agent contains, based on the total weight of the agent, one or more persulfates from the group ammonium peroxodisulfate, potassium peroxodisulfate and sodium peroxodisulfate in a total amount of from 0.5 to 20 wt.%, preferably from 1.0 to 12.5 wt.%, more preferably from 2.5 to 10 wt.% and particularly preferably from 3.0 to 6.0 wt.%.

10. The agent according to one of claims 1 to 9, **characterized in that** said agent contains, based on the total weight of the agent,
- 0.2 wt.% to 13.5 wt.%, preferably 0.5 wt.% to 1.5 wt.% ammonium peroxodisulfate
- 0.5 wt.% to 4.5 wt.%, preferably 1.5 wt.% to 2.5 wt.% potassium peroxodisulfate and
- 0.2 wt.% to 1.8 wt.%, preferably 0.4 wt.% to 0.8 wt.% sodium peroxodisulfate.

11. The agent according to one of claims 1 to 10, **characterized in that** said agent contains at least one SiO₂ compound from the group of silicic acids, silicates, water glasses and/or alkali metal metasilicates.

12. The agent according to one of claims 1 to 11, **characterized in that** said agent additionally contains at least one further cationic substantive dye which differs from the dyes of formula (I).

13. The agent according to one of claims 1 to 12, **characterized in that** said agent additionally contains Basic Orange 31 and/or Basic Red 51.

## Revendications

1. Agent de modification de la couleur de fibres kératiniques, en particulier de cheveux humains, contenant dans un support cosmétique
(a) au moins un colorant à action directe de formule (I), dans laquelle 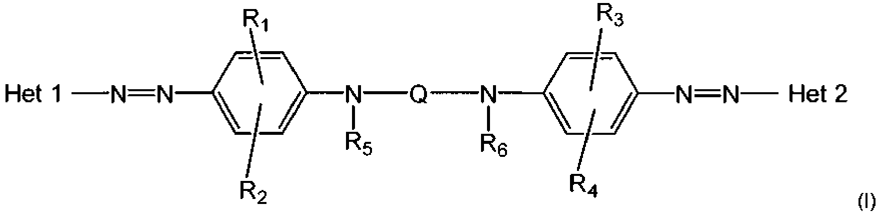
Het1 et Het2 représentent, indépendamment l'un de l'autre, l'une des structures (II), (III), (IV), (V), (VI) ou (VII), 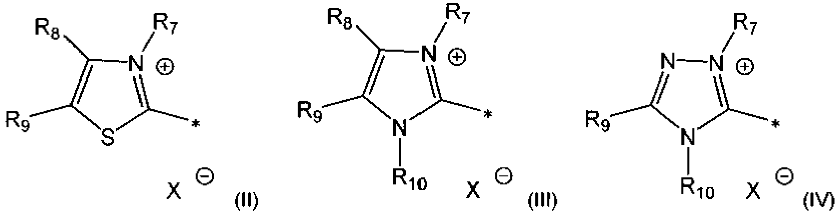 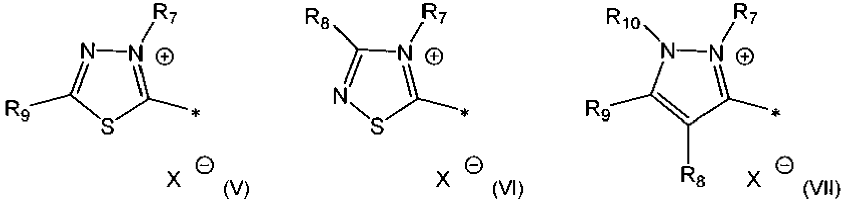
R1 et R3 représentent, indépendamment l'un de l'autre, un groupe alkyle en C₁ à C₆ ou un groupe alcoxy en C₁ à C₆,
R2 et R4 représentent tous deux un atome d'hydrogène,
R5 et R6 représentent, indépendamment l'un de l'autre, un atome d'hydrogène, un groupe alkyle en C₁ à C₆ ou un groupe alcényle en C₂ à C₆,
Q représente un groupement de formule (VIII), *-(CH₂)n-*
n représente un nombre entier compris entre 3 et 6,
R7 et R10 représentent chacun, indépendamment l'un de l'autre, un groupe alkyle en C₁ à C₆ ou un groupe alcényle en C₂ à C₆,
R8 et R9 représentent chacun, indépendamment l'un de l'autre, un atome d'hydrogène, un groupe alkyle en C₁ à C₆, un atome d'halogène choisi dans le groupe comprenant le chlore, le brome, le fluor ou l'iode, un groupe alcoxy en C₁ à C₆ ou un groupe nitro,
X- représente un anion physiologiquement compatible, de préférence dans le groupe comprenant le chlorure, le bromure, l'iodure, le méthylsulfate, le méthylsulfonate, le p-toluènesulfonate, l'acétate, le sulfate d'hydrogène, un demi-sulfate ou un demi-tétrachlorozincate.

2. Agent selon la revendication 1, **caractérisé en ce qu'**il contient (a) au moins un colorant à action directe de formule générale (I), dans laquelle
Het1 et Het2 représentent chacun, indépendamment l'un de l'autre, l'une des structures (II), (III) ou (IV).

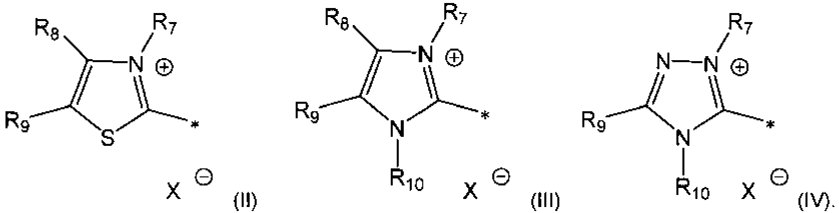

3. Agent selon l'une des revendications 1 à 2, **caractérisé en ce qu'**il contient (a) au moins un colorant à action directe de formule générale (I), dans laquelle
R5 et R6 représentent, indépendamment l'un de l'autre, l'hydrogène ou un groupe alkyle en C₁-C₆.

4. Agent selon l'une des revendications 1 à 3, **caractérisé en ce qu'**il contient (a) au moins un colorant à action directe de formule générale (I), dans laquelle
R7 et R10 représentent chacun, indépendamment l'un de l'autre, un groupe méthyle ou un groupe éthyle,
R8 et R9 représentent un atome d'hydrogène.

5. Agent selon l'une des revendications 1 à 4, **caractérisé en ce qu'**il contient (a) au moins un colorant à action directe de formule générale (I), choisi parmi les
- sels du 3-méthyl-2-[2-(3-méthyl-4-{[3-({2-méthyl-4-[2-(3-méthyl-1,3-thiazol-3-ium-2-yl)diazen-1-yl]phényl}amino)propyl]amino}phényl)diazen-1-yl]-1,3-thiazol-3-ium
- sels du 3-méthyl-2-[2-(2-méthyl-4-{[3-({3-méthyl-4-[2-(3-méthyl-1,3-thiazol-3-ium-2-yl)diazen-1-yl]phényl}amino)propyl]amino}phényl)diazen-1-yl]-1,3-thiazol-3-ium
- sels du 3-méthyl-2-[2-(3-méthyl-4-{[4-({2-méthyl-4-[2-(3-méthyl-1,3-thiazol-3-ium-2-yl)diazen-1-yl]phényl}amino)butyl]amino}phényl)diazen-1-yl]-1,3-thiazol-3-ium
- sels du 3-méthyl-2-[2-(2-méthyl-4-{[4-({3-méthyl-4-[2-(3-méthyl-1,3-thiazol-3-ium-2-yl)diazen-1-yl]phényl}amino)butyl]amino}phényl)diazen-1-yl]-1,3-thiazol-3-ium
- sels du 3-méthyl-2-[2-(3-méthyl-4-{[5-({2-méthyl-4-[2-(3-méthyl-1,3-thiazol-3-ium-2-yl)diazen-1-yl]phényl}amino)pentyl]amino}phényl)diazen-1-yl]-1,3-thiazol-3-ium
- sels du 3-méthyl-2-[2-(2-méthyl-4-{[5-({3-méthyl-4-[2-(3-méthyl-1,3-thiazol-3-ium-2-yl)diazen-1-yl]phényl}amino)pentyl]amino}phényl)diazen-1-yl]-1,3-thiazol-3-ium
- sels du 3-méthyl-2-(2-{3-méthyl-4-[méthyl({3-[méthyl({2-méthyl-4-[2-(3-méthyl-1,3-thiazol-3-ium-2-yl)diazen-1-yl]phényl})amino]propyl})amino]phényl}diazen-1-yl)-1,3-thiazol-3-ium
- sels du 3-méthyl-2-(2-{3-méthyl-4-[éthyl({3-[éthyl({2-méthyl-4-[2-(3-méthyl-1,3-thiazol-3-ium-2-yl)diazen-1-yl]phényl})amino]propyl})amino]phényl}diazen-1-yl)-1,3-thiazol-3-ium
- sels du 3-méthyl-2-(2-{2-méthyl-4-[méthyl({3-[méthyl({3-méthyl-4-[2-(3-méthyl-1,3-thiazol-3-ium-2-yl)diazen-1-yl]phényl})amino]propyl})amino]phényl}diazen-1-yl)-1,3-thiazol-3-ium
- sels du 3-méthyl-2-(2-{2-méthyl-4-[éthyl({3-[éthyl({3-méthyl-4-[2-(3-méthyl-1,3-thiazol-3-ium-2-yl)diazen-1-yl]phényl})amino]propyl})amino]phényl}diazen-1-yl)-1,3-thiazol-3-ium
- sels du 3-méthyl-2-(2-{3-méthyl-4-[méthyl({4-[méthyl({2-méthyl-4-[2-(3-méthyl-1,3-thiazol-3-ium-2-yl)diazen-1-yl]phényl})amino]butyl})amino]phényl}diazen-1-yl)-1,3-thiazol-3-ium
- sels du 3-méthyl-2-(2-{2-méthyl-4-[méthyl({4-[méthyl({3-méthyl-4-[2-(3-méthyl-1,3-thiazol-3-ium-2-yl)diazen-1-yl]phényl})amino]butyl})amino]phényl}diazen-1-yl)-1,3-thiazol-3-ium
- sels du 3-méthyl-2-(2-{3-méthyl-4-[méthyl({5-[méthyl({2-méthyl-4-[2-(3-méthyl-1,3-thiazol-3-ium-2-yl)diazen-1-yl]phényl})amino]pentyl})amino]phényl}diazen-1-yl)-1,3-thiazol-3-ium
- sels du 3-méthyl-2-(2-{2-méthyl-4-[méthyl({5-[méthyl({3-méthyl-4-[2-(3-méthyl-1,3-thiazol-3-ium-2-yl)diazen-1-yl]phényl})amino]pentyl})amino]phényl}diazen-1-yl)-1,3-thiazol-3-ium
- sels du 2-[2-(3-méthoxy-4-{[3-({2-méthoxy-4-[2-(3-méthyl-1,3-thiazol-3-ium-2-yl)diazen-1-yl]phényl}amino)propyl]amino}phényl)diazen-1-yl]-3-méthyl-1,3-thiazol-3-ium
- sels du 2-[2-(2-méthoxy-4-{[3-({3-méthoxy-4-[2-(3-méthyl-1,3-thiazol-3-ium-2-yl)diazen-1-yl]phényl}amino)propyl]amino}phényl)diazen-1-yl]-3-méthyl-1,3-thiazol-3-ium
- sels du 2-[2-(3-méthoxy-4-{[4-({2-méthoxy-4-[2-(3-méthyl-1,3-thiazol-3-ium-2-yl)diazen-1-yl]phényl}amino)butyl]amino}phényl)diazen-1-yl]-3-méthyl-1,3-thiazol-3-ium
- sels du 2-[2-(2-méthoxy-4-{[4-({3-méthoxy-4-[2-(3-méthyl-1,3-thiazol-3-ium-2-yl)diazen-1-yl]phényl}amino)butyl]amino}phényl)diazen-1-yl]-3-méthyl-1,3-thiazol-3-ium
- sels du 2-[2-(3-méthoxy-4-{[5-({2-méthoxy-4-[2-(3-méthyl-1,3-thiazol-3-ium-2-yl)diazen-1-yl]phényl}amino)pentyl]amino}phényl)diazen-1-yl]-3-méthyl-1,3-thiazol-3-ium
- sels du 2-[2-(2-méthoxy-4-{[5-({3-méthoxy-4-[2-(3-méthyl-1,3-thiazol-3-ium-2-yl)diazen-1-yl]phényl}amino)pentyl]amino}phényl)diazen-1-yl]-3-méthyl-1,3-thiazol-3-ium
- sels du 2-[2-(3-méthoxy-4-{[3-({2-méthoxy-4-[2-(3-méthyl-1,3-thiazol-3-ium-2-yl)diazen-1-yl]phényl}(méthyl)amino)propyl](méthyl)amino}phényl)diazen-1-yl]-3-méthyl-1,3-thiazol-3-ium
- sels du 2-[2-(2-méthoxy-4-{[3-({3-méthoxy-4-[2-(3-méthyl-1,3-thiazol-3-ium-2-yl)diazen-1-yl]phényl}(méthyl)amino)propyl](méthyl)amino}phényl)diazen-1-yl]-3-méthyl-1,3-thiazol-3-ium
- sels du 2-[2-(3-méthoxy-4-{[4-({2-méthoxy-4-[2-(3-méthyl-1,3-thiazol-3-ium-2-yl)diazen-1-yl]phényl}(méthyl)amino)butyl](méthyl)amino}phényl)diazen-1-yl]-3-méthyl-1,3-thiazol-3-ium
- sels du 2-[2-(2-méthoxy-4-{[4-({3-méthoxy-4-[2-(3-méthyl-1,3-thiazol-3-ium-2-yl)diazen-1-yl]phényl}(méthyl)amino)butyl](méthyl)amino}phényl)diazen-1-yl]-3-méthyl-1,3-thiazol-3-ium
- sels du 2-[2-(3-méthoxy-4-{[5-({2-méthoxy-4-[2-(3-méthyl-1,3-thiazol-3-ium-2-yl)diazen-1-yl]phényl}(méthyl)amino)pentyl](méthyl)amino}phényl)diazen-1-yl]-3-méthyl-1,3-thiazol-3-ium
- sels du 2-[2-(2-méthoxy-4-{[5-({3-méthoxy-4-[2-(3-méthyl-1,3-thiazol-3-ium-2-yl)diazen-1-yl]phényl}(méthyl)amino)pentyl](méthyl)amino}phényl)diazen-1-yl]-3-méthyl-1,3-thiazol-3-ium
- sels du 5-[2-(4-{[3-({4-[2-(1,4-diméthyl-1H-1,2,4-triazol-4-ium-5-yl)diazen-1-yl]-2-méthylphényl}amino)propyl]amino}-3-méthylphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-3-méthylphényl}amino)propyl]amino}-2-méthylphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-2-méthylphényl}amino)butyl]amino}-3-méthylphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-3-méthylphényl}amino)butyl]amino}-2-méthylphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-2-méthylphényl}amino)pentyl]amino}-3-méthylphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-3-méthylphényl}amino)pentyl]amino}-2-méthylphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-2-méthylphényl}(méthyl)amino)propyl](méthyl)amino}-3-méthylphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-3-méthylphényl}(méthyl)amino)propyl](méthyl)amino}-2-méthylphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-2-méthylphényl}(méthyl)amino)butyl](méthyl)amino}-3-méthylphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-3-méthylphényl}(méthyl)amino)butyl](méthyl)amino}-2-méthylphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-2-méthylphényl}(méthyl)amino)pentyl](méthyl)amino}-3-méthylphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-3-méthylphényl}(méthyl)amino)pentyl](méthyl)amino}-2-méthylphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-2-méthoxyphényl}amino)propyl]amino}-3-méthoxyphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-3-méthoxyphényl}amino)propyl]amino}-2-méthoxyphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-2-méthoxyphényl}amino)butyl]amino}-3-méthoxyphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-3-méthoxyphényl}amino)butyl]amino}-2-méthoxyphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-2-méthoxyphényl}amino)pentyl]amino}-3-méthoxyphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-3-méthoxyphényl}amino)pentyl]amino}-2-méthoxyphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-2-méthoxyphényl}(méthyl)amino)propyl](méthyl)amino}-3-méthoxyphényl)diazen-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazen-1-yl]-3-méthoxyphényl}(méthyl)amino)propyl](méthyl)amino}-2-méthoxyphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-2-méthoxyphényl}(méthyl)amino)butyl](méthyl)amino}-3-méthoxyphényl)diazen-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazen-1-yl]-3-méthoxyphényl}(méthyl)amino)butyl](méthyl)amino}-2-méthoxyphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-2-méthoxyphényl}(méthyl)amino)pentyl](méthyl)amino}-3-méthoxyphényl)diazen-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazen-1-yl]-3-méthoxyphényl}(méthyl)amino)pentyl](méthyl)amino}-2-méthoxyphényl)diazen-1-yl]-1,4-diméthyl-1H-1,2,4-triazol-4-ium

6. Agent selon l'une des revendications 1 à 5, **caractérisé en ce qu'**il contient, par rapport au poids total de l'agent, un ou plusieurs colorants à action directe (a) de formule (I) en une quantité totale de 0,01 à 4,5 % en poids, de préférence de 0,05 à 2,8 % en poids, de manière davantage préférée de 0,1 à 2,2 % en poids et de manière particulièrement préférée de 0,2 à 1,2 % en poids.

7. Agent selon l'une des revendications 1 à 6, **caractérisé en ce qu'**il contient, par rapport au poids total de l'agent, de 0,5 à 12,5 % en poids, de préférence de 2,5 à 10 % en poids et en particulier de 4,0 à 9,0 % en poids de peroxyde d'hydrogène.

8. Agent selon l'une des revendications 1 à 7, **caractérisé en ce qu'**il contient au moins un persulfate du groupe constitué du peroxodisulfate d'ammonium, du peroxodisulfate de potassium et du peroxodisulfate de sodium.

9. Agent selon l'une des revendications 1 à 8, **caractérisé en ce qu'**il contient, par rapport au poids total de l'agent, un ou plusieurs persulfates du groupe constitué du peroxodisulfate d'ammonium, du peroxodisulfate de potassium et du peroxodisulfate de sodium en une quantité totale de 0,5 à 20 % en poids, de préférence de 1,0 à 12,5 % en poids, de manière davantage préférée de 2,5 à 10 % en poids et de manière particulièrement préférée de 3,0 à 6,0 % en poids.

10. Agent selon l'une des revendications 1 à 9, **caractérisé en ce qu'**il contient, par rapport au poids total de l'agent,
- de 0,2 à 13,5 % en poids, de préférence de 0,5 à 1,5 % en poids, de peroxodisulfate d'ammonium,
- de 0,5 à 4,5 % en poids, de préférence de 1,5 à 2,5 % en poids, de peroxodisulfate de potassium, et
- de 0,2 à 1,8 % en poids, de préférence de 0,4 à 0,8 % en poids, de peroxodisulfate de sodium.

11. Agent selon l'une des revendications 1 à 10, **caractérisé en ce qu'**il contient au moins un composé SiO₂ du groupe des silices, des silicates, des verres solubles et/ou des métasilicates alcalins.

12. Agent selon l'une des revendications 1 à 11, **caractérisé en ce qu'**il contient en outre au moins un autre colorant cationique à action directe, lequel est différent des colorants de la formule (I).

13. Agent selon l'une des revendications 1 à 12, **caractérisé en ce qu'**il contient en outre de l'orange basique 31 et/ou du rouge basique 51.
